(19) Europäisches Patentamt
European Patent Office
Office européen des brevets

(11) **EP 2 719 408 A1**

(12) **EUROPEAN PATENT APPLICATION**
published in accordance with Art. 153(4) EPC

(43) Date of publication:
**16.04.2014 Bulletin 2014/16**

(21) Application number: **12796917.8**

(22) Date of filing: **06.06.2012**

(51) Int Cl.:
***A61M 1/18*** (2006.01) ***B01D 63/02*** (2006.01)
***B01D 69/08*** (2006.01) ***B01D 71/44*** (2006.01)
***B01D 71/68*** (2006.01) ***D01F 6/00*** (2006.01)
***D01F 6/76*** (2006.01) ***D01F 6/94*** (2006.01)

(86) International application number:
**PCT/JP2012/064561**

(87) International publication number:
**WO 2012/169529 (13.12.2012 Gazette 2012/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2011 JP 2011129437**
**04.07.2011 JP 2011148534**

(71) Applicant: **Asahi Kasei Medical Co., Ltd.**
**Chiyoda-ku**
**Tokyo 101-8101 (JP)**

(72) Inventors:
- **SATOH, Junya**
**Tokyo 101-8101 (JP)**
- **MORI, Kouichirou**
**Tokyo 101-8101 (JP)**

(74) Representative: **von Kreisler Selting Werner**
**Deichmannhaus am Dom**
**Bahnhofsvorplatz 1**
**50667 Köln (DE)**

# (54) HOLLOW FIBER MEMBRANE FOR BLOOD TREATMENT AND HOLLOW FIBER MEMBRANE-TYPE BLOOD TREATMENT APPARATUS

(57) [Problem to be Solved]

It is intended to obtain a hollow fiber membrane for blood processing that has high membrane performance, can reduce leak misdetection, is excellent in antioxidative performance and blood compatibility, carries a low risk of hydrophilic polymer elution or a leak from hollow fibers, is easy to prime, and has highly stable permeability in a harsh environment.

[Solution]

The present invention provides a hollow fiber membrane for blood processing, containing a hydrophilic polymer, a polysulfone-based resin, and a fat-soluble vitamin, wherein
a content A of the hydrophilic polymer, which is a mass ratio of the hydrophilic polymer to a total mass of the hydrophilic polymer and the polysulfone-based resin in a whole of the hollow fiber membrane for blood processing, is 3% by mass or larger and 10% by mass or smaller,
an abundance ratio B of the hydrophilic polymer, which is a mass ratio of the hydrophilic polymer to the total mass of the hydrophilic polymer and the polysulfone-based resin in an inner surface of the hollow fiber membrane for blood processing, is 35% by mass or larger and 50% by mass or smaller, and
an abundance of the fat-soluble vitamin in an entire surface of the hollow fiber membrane for blood processing is 0.5 mg or larger and 25 mg or smaller based on 1 g of the hollow fiber membrane.

FIG. 1

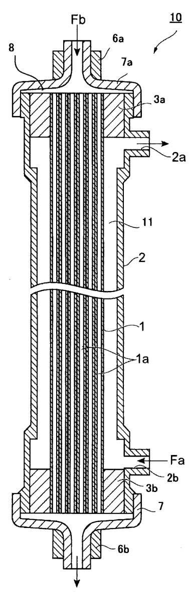


EP 2 719 408 A1

## Description

Technical Field

The present invention relates to a hollow fiber membrane for blood processing and a hollow fiber membrane-based blood processing apparatus.

Background Art

Heretofore, hollow fiber membrane-based blood processing apparatuses using selectively permeable membranes have been used widely in the field of extracorporeal blood circulation, for example, hemodialysis, oxygen supply to blood during open-heart surgery, or plasma separation.

In recent years, hollow fiber membranes for blood processing (hereinafter, also simply referred to as hollow fiber membranes) made of polysulfone-based resins (hereinafter, also referred to as polysulfones) as materials have been used widely, particularly, in the field of blood processing membranes such as dialysis membranes, gas exchange membranes, and blood component separation membranes.

The hollow fiber membranes for blood processing made of highly hydrophobic polysulfones alone as materials produce insufficient blood compatibility. For this reason, complexes of the polysulfones with hydrophilic polymers such as polyvinylpyrrolidone are generally used.

Attempts have also been made to impart a role in alleviating oxidative stress observed in patients receiving long-term dialysis, to the hollow fiber membranes for blood processing already taking a role as separation membranes. Possible approaches are, for example, to eliminate peroxide causative of oxidative stress by use of the hollow fiber membranes for blood processing and to allow organisms to recover antioxidative effect. Specifically, there has been proposed a hollow fiber membrane-based blood processing apparatus provided with a hollow fiber membrane for blood processing in which the surface of a dialysis membrane is covered with vitamin E having various physiological effects such as *in vivo* antioxidative effect, biomembrane stabilizing effect, and platelet aggregation inhibitory effect (see e.g., Patent Document 1).

Clinical studies have reported that use of a hollow fiber membrane-based blood processing apparatus equipped with a vitamin E-immobilized hollow fiber membrane for blood processing can reduce the dose of a erythropoiesis stimulating agent which is indispensable for dialysis treatment and, at the same time, is very expensive (see e.g., Non-Patent Documents 1 and 2). These pieces of information suggest that the proliferation of a fat-soluble vitamin-immobilized hollow fiber membrane-based blood processing apparatus is preferred in terms of medical economy.

Of course, various hollow fiber membrane-based blood processing apparatuses as described above are completely sterilized under hermetically sealed package conditions before use in consideration of their use application. For example, gaseous sterilization using ethylene oxide gas or the like, autoclaving using high-pressure steam, and radiation sterilization using $\gamma$-rays, electron beam, or the like have heretofore been known as methods for sterilizing the hollow fiber membrane-based blood processing apparatuses. Of these methods, the gaseous sterilization method using ethylene oxide gas may be harmful to human bodies due to undesirable residues of ethylene oxide gas. Alternatively, the autoclaving method using high-pressure steam may significantly reduce the performance of the hollow fiber membrane-based blood processing apparatus during sterilization, depending on materials constituting the apparatus.

It has also been pointed out that sterilization by the autoclaving method using high-pressure steam, when applied to a vitamin E-immobilized polysulfone membrane, may cause the local aggregation of the fat-soluble vitamin and generate cracks in the hollow fiber membrane, resulting in the increased possibility of a blood leak (see e.g., Patent Document 2).

By contrast, the radiation sterilization method is free from these problems, i.e., the residues of ethylene oxide gas or a leak from the hollow fiber membrane, and is thus preferred for sterilization.

These hollow fiber membrane-based blood processing apparatuses are broadly classified into: wet type in which the hollow interior of the hollow fiber membrane for blood processing or the gap between the hollow fiber membrane and a container is filled with an aqueous medium; and a dry type in which such a space is not filled with an aqueous medium.

The hollow fiber membrane-based blood processing apparatuses of this dry type can be further divided into a type having a membrane with a small percent or lower of water content (dry type in the narrow sense) and a type having a membrane moderately wetted with water, a humectant, or the like. Although the latter is also called semidry type differentiated from the dry type in the narrow sense, these types have substantially the same features and are therefore collectively referred to as dry type in the present specification. The features of the hollow fiber membrane-based blood processing apparatuses of dry type are that the products have a smaller weight than that of wet type and are hardly frozen at low temperatures. Thus, the hollow fiber membrane-based blood processing apparatuses of dry type are excellent in terms of distribution including transportation and storage.

It is however known that these hollow fiber membrane-based blood processing apparatuses of dry type exhibit

reduction in blood compatibility upon radiation sterilization, because the hydrophilic polymer constituting the hollow fiber membrane for blood processing is degraded or eluted. In light of this problem, there has been proposed a method for suppressing reduction in blood compatibility, which involves protecting a hollow fiber membrane with a particular amount of a wet protectant against radiation sterilization (see e.g., Patent Document 2), or which involves protecting a hollow fiber membrane with a particular amount of a wet protectant and controlling its neighboring oxygen concentration, followed by sterilization with electron beam (see e.g., Patent Document 3).

If the hollow fiber membrane-based blood processing apparatus has a pinhole (defect) in its hollow fiber membrane, an undesired substance may penetrate the hollow fiber membrane. In this respect, a leak test to confirm the presence or absence of a pinhole in the hollow fiber membrane is required in the course of assembly of the hollow fiber membrane-based blood processing apparatus.

For example, in the case of a low gas-permeable hollow fiber membrane, one of spaces isolated therebetween by the hollow fiber membrane is pressurized with gas, and the rate at which the gas gets into the other space can be measured to determine the presence or absence of a pinhole in the hollow fiber membrane.

In the case of a recent hollow fiber membrane-based blood processing apparatus using a highly water-permeable hollow fiber membrane, however, the presence or absence of a pinhole is difficult to determine by the method described above, because the hollow fiber membrane is very highly permeable to gas. Specifically, this phenomenon is evident in a highly permeable membrane having an ultrafiltration rate (UFR) (which will be described later in detail in Examples) exceeding 40 mL/(hr·mmHg), and presents a great obstacle to the achievement of high membrane performance.

Thus, a method which involves temporarily wetting the hollow fiber membrane with a liquid to make the membrane almost impermeable to gas in the absence of a pinhole, followed by the leak test (see e.g., Patent Document 4) and a method which involves completely dipping the hollow fiber membrane in a liquid, then in this state, pressuring the hollow portion of the hollow fiber membrane with gas, and detecting the presence or absence of a pinhole on the basis of the presence or absence of bubbles coming from the hollow fiber membrane (see e.g., Patent Document 5) have been practiced in order to conduct the leak test of the hollow fiber membrane having high water permeability and high gas permeability. Also, a leak inspection method for hollow fiber membrane modules, which involves introducing gas containing colored particles to the inside of the hollow fiber membrane under pressure and detecting a leak of the colored particles (see e.g., Patent Document 6) has also been proposed as an alternative leak test.

List of Prior Art

Patent Document

Patent Document 1: Japanese Patent Laid-Open No. 7-178166
Patent Document 2: Japanese Patent Laid-Open No. 2006-296931
Patent Document 3: Japanese Patent Laid-Open No. 2008-93228
Patent Document 4: Japanese Patent Laid-Open No. 2005-238096
Patent Document 5: Japanese Patent Laid-Open No. 2003-190747
Patent Document 6: Japanese Patent Laid-Open No. 2009-183822

Non-Patent Document

Non-Patent Document 1: Andrulli S et al., Nephron Clin Pract. 2010, 115: c82-89
Non-Patent Document 2: Panichi V et al., Blood Purification. 2011 32: 7-14

Summary of Invention

Problems To Be Solved By The Invention

Patent Documents 4 to 6 described above disclose various approaches as to methods for conducting a leak test on a highly gas-permeable hollow fiber membrane. These approaches, however, are still susceptible to improvement. Specifically, a hollow fiber membrane-based blood processing apparatus actually having no pinhole may be mistakenly determined to have a pinhole in the leak test (this is referred to as "leak misdetection" in the present specification) even if the hollow fiber membrane is wetted with a liquid. Such leak misdetection leads to the disposal of the apparatus and is thus responsible for great reduction in production efficiency. An approach for reliably conducting a leak test with leak

misdetection reduced has been unknown, particularly, as to a highly gas-permeable hollow fiber membrane applied to a hollow fiber membrane-based blood processing apparatus using a fat-soluble vitamin for the purpose of alleviating oxidative stress, as disclosed in Patent Document 1 or 2.

Blood purification therapy has been being spread worldwide in recent years and is becoming available not only in Japan or the United States and European countries where this therapy is currently used dominantly, but in various regions such as China, India, Russia, Middle Eastern countries, Latin American countries, and south-eastern Asia. Hollow fiber membrane-based blood processing apparatuses used in such a wide variety of countries are managed on various conditions as medical equipment. The stability assurance of 3-year storage in a normal-temperature and normal-humidity environment is not adequate, though this is currently recognized as a general storage form.

For example, when a cargo container accommodating the hollow fiber membrane-based blood processing apparatus is exposed to direct sunlight in a container yard, its internal temperature is likely to rise to 60°C or higher. Alternatively, the hollow fiber membrane-based blood processing apparatus may be left over a long time in an unexpected high-temperature environment, for example, due to extended transportation by truck in a large country such as the United States or transportation by ship travelling in the tropical zone (equator). When the conventional hollow fiber membranes for blood processing as described above are exposed to such an unexpected harsh environment, their various characteristics, particularly, permeability may be degraded.

Also, a medical device provided with a hollow fiber membrane for blood processing having a much higher level of blood compatibility will be required.

The present inventors, however, have conducted a blood compatibility model test aimed at a higher level of blood compatibility and consequently revealed that the hollow fiber membrane for blood processing disclosed in Patent Document 3 still produces practically insufficient blood compatibility and is susceptible to further improvement. Patent Document 3 exemplifies vitamin E as a wet protectant for protecting the hollow fiber membrane. When the hollow fiber membrane is wetted with vitamin E in the range disclosed in this Patent Document 3, the vitamin E (fat-soluble vitamin), which cannot be removed by priming, clogs fine pores in the surface of the hollow fiber membrane due to too strong influence of hydrophobicity of the vitamin E. Unfortunately, its functions as a blood processing membrane are therefore inhibited.

A vitamin E-immobilized polysulfone membrane having a practical rate of impregnation with vitamin E (wet protectant) as a blood processing membrane has been shown to still produce practically insufficient blood compatibility after being radiation-sterilized in a dry state.

Further studies conducted by the present inventors have showed that: an attempt to secure blood compatibility after radiation sterilization by increasing the content of a hydrophilic polymer responsible for blood compatibility in the hollow fiber membrane still results in insufficient blood compatibility; and in addition, the resulting hollow fiber membrane is poorly practical in such a way that the hollow fiber membrane carries a risk of hydrophilic polymer elution or causes a leak by vibration or the like during transportation or storage due to reduced membrane strength, i.e., is at an increased risk of breakage attributed to insufficient strength of the hollow fiber membrane, or air cannot be completely removed by priming before use.

The conventional hollow fiber membrane-based blood processing apparatuses have insufficiently stable permeability in a harsh environment, as described above.

For these reasons, there is a strong demand for a hollow fiber membrane for blood processing that maintains high membrane performance, i.e., water permeability and gas permeability, while it can reduce leak misdetection, has excellent antioxidative performance and blood compatibility, carries a low risk of hydrophilic polymer elution or a leak from the hollow fiber membrane, is easy to prime, and has highly stable permeability even in a harsh environment, and a hollow fiber membrane-based blood processing apparatus provided with the hollow fiber membrane.

Thus, an object of the present invention is to provide a hollow fiber membrane for blood processing that maintains high membrane performance while it can reduce leak misdetection, has excellent antioxidative performance and blood compatibility, carries a low risk of hydrophilic polymer elution or a leak from the hollow fiber membrane, is easy to prime, and has highly stable permeability in a harsh environment, and a hollow fiber membrane-based blood processing apparatus provided with the hollow fiber membrane.

## Means For Solving The Problems

The present inventors have conducted diligent studies to attain the object and consequently completed the present invention by finding that, in a hollow fiber membrane containing a polysulfone-based resin, a hydrophilic polymer, and a fat-soluble vitamin, the content of the hydrophilic polymer in a whole of the hollow fiber membrane, an abundance ratio of the hydrophilic polymer in an inner surface of the hollow fiber membrane, and an abundance of the fat-soluble vitamin in an entire surface of the hollow fiber membrane can be set to particular values, which are effective for obtaining a hollow fiber membrane for blood processing that maintains high membrane performance while it can reduce leak misdetection, has excellent antioxidative performance and blood compatibility, carries a low risk of hydrophilic polymer

elution or a leak from the hollow fiber membrane, is easy to prime, and has highly stable permeability even in a harsh environment, and a hollow fiber membrane-based blood processing apparatus provided with the hollow fiber membrane.

Specifically, the present invention is as follows:

[1] A hollow fiber membrane for blood processing, comprising a hydrophilic polymer, a polysulfone-based resin, and a fat-soluble vitamin, wherein
a content A of the hydrophilic polymer, which is a mass ratio of the hydrophilic polymer to a total mass of the hydrophilic polymer and the polysulfone-based resin in a whole of the hollow fiber membrane for blood processing, is 3% by mass or larger and 10% by mass or smaller,
an abundance ratio B of the hydrophilic polymer, which is a mass ratio of the hydrophilic polymer to the total mass of the hydrophilic polymer and the polysulfone-based resin in an inner surface of the hollow fiber membrane for blood processing, is 35% by mass or larger and 50% by mass or smaller, and
an abundance of the fat-soluble vitamin in an entire surface of the hollow fiber membrane for blood processing is 0.5 mg or larger and 25 mg or smaller based on 1 g of the hollow fiber membrane.
[2] The hollow fiber membrane for blood processing according to item [1] above, wherein the hydrophilic polymer is polyvinylpyrrolidone or polyethylene glycol.
[3] The hollow fiber membrane for blood processing according to item [1] or [2] above, wherein the hollow fiber membrane for blood processing comprises 1 ppm or higher and 8 ppm or lower nitric acid ions.
[4] A hollow fiber membrane-based blood processing apparatus provided with a hollow fiber membrane for blood processing according to any one of items [1] to [3] above inside a container.

Advantageous Effects of Invention

The present invention can produce a polysulfone-based hollow fiber membrane for blood processing that maintains high membrane performance while it can reduce leak misdetection, has excellent antioxidative performance and blood compatibility, carries a low risk of hydrophilic polymer elution or a leak from the hollow fiber membrane, is easy to prime, and has highly stable permeability even in a harsh environment, and a hollow fiber membrane-based blood processing apparatus provided with the hollow fiber membrane.

Brief Description of Drawings

[Figure 1] Figure 1 is a schematic cross-sectional view of the hollow fiber membrane-based blood processing apparatus according to the present embodiment.
[Figure 2] Figure 2 is a schematic cross-sectional view of a surface of a hollow fiber membrane constituting the hollow fiber membrane-based blood processing apparatus in a state before radiation sterilization.
[Figure 3] Figure 3 is a schematic cross-sectional view of a surface of a hollow fiber membrane radiation-sterilized in a dry state.
[Figure 4] Figure 4 is a schematic cross-sectional view of the surface of a hollow fiber membrane before radiation sterilization, wherein the hollow fiber membrane contains additional hydrophilic polymers immobilized after its formation.
[Figure 5] Figure 5 is a schematic cross-sectional view of a surface of a hollow fiber membrane radiation-sterilized in a dry state, wherein the hollow fiber membrane contains additional hydrophilic polymers immobilized after its formation.
[Figure 6] Figure 6 is a schematic cross-sectional view of a surface of a fat-soluble vitamin-containing hollow fiber membrane in a state before radiation sterilization, wherein the hollow fiber membrane contains additional hydrophilic polymers immobilized after its formation.
[Figure 7] Figure 7 is a schematic cross-sectional view of a surface of a fat-soluble vitamin-containing hollow fiber membrane radiation-sterilized in a dry state, wherein the hollow fiber membrane contains additional hydrophilic polymers immobilized after its formation.
[Figure 8] Figure 8 is a conceptual diagram of a surface of a hollow fiber membrane consisting only of a polysulfone-based resin and a hydrophilic polymer.
[Figure 9] Figure 9 is a flow chart of a method for producing a hollow fiber membrane for blood processing.
[Figure 10] Figure 10 is a schematic view showing a method for producing the hollow fiber membrane for blood processing according to the present embodiment.
[Figure 11] Figure 11 is a graph showing the relationship between the amount of the fat-soluble vitamin in an entire surface of the hollow fiber membrane and blood compatibility.

[Figure 12] Figure 12 is a graph showing the relationship between an abundance ratio B of the hydrophilic polymer in an inner surface of the hollow fiber membrane and blood compatibility.
[Figure 13] Figure 13 is a schematic view of an apparatus for measuring the residual quantity of air.
[Figure 14] Figure 14 is a schematic view of an apparatus for measuring an ultrafiltration rate (UFR).

Mode For Carrying Out The Invention

Hereinafter, a mode for carrying out the present invention (hereinafter, referred to as the "present embodiment") will be described in detail with reference to the drawings. The present invention is not limited by the description below, and various changes or modifications can be made therein without departing from the spirit of the present invention.
In each drawing, positional relationships indicated by the terms "upper", "lower", "right", "left", and the like are based on those shown in the drawing, unless otherwise specified. In addition, the dimensional ratios of each drawing are not intended to be limited to those shown in the drawing.

[Hollow fiber membrane for blood processing]

The hollow fiber membrane for blood processing of the present embodiment is

a hollow fiber membrane for blood processing, comprising a hydrophilic polymer, a polysulfone-based resin, and a fat-soluble vitamin, wherein a content A of the hydrophilic polymer, which is a mass ratio of the hydrophilic polymer to a total mass of the hydrophilic polymer and the polysulfone-based resin in the whole of the hollow fiber membrane for blood processing, is 3% by mass or larger and 10% by mass or smaller,
an abundance ratio B of the hydrophilic polymer, which is a mass ratio of the hydrophilic polymer to the total mass of the hydrophilic polymer and the polysulfone-based resin in an inner surface of the hollow fiber membrane for blood processing, is 35% by mass or larger and 50% by mass or smaller, and
an abundance of the fat-soluble vitamin in the entire surface of the hollow fiber membrane for blood processing is 0.5 mg or larger and 25 mg or smaller based on 1 g of the hollow fiber membrane.

In this context, the inner surface of the hollow fiber membrane for blood processing refers to the outermost surface on the inner side of the hollow fiber membrane, i.e., a hollow fiber membrane surface with which blood is contacted.
The entire surface of the hollow fiber membrane for blood processing includes the inner surface of the hollow fiber membrane with which blood is directly contacted as well as its outer surface and the inner surfaces of pores in a porous portion constituting the thickness of the membrane.
The hollow fiber membrane for blood processing according to the present embodiment is housed in a predetermined container to constitute a hollow fiber membrane-based blood processing apparatus.
The hollow fiber membrane-based blood processing apparatus generally has a structure shown in Figure 1 and may have any of other structures as long as the apparatus has an inlet and an outlet for blood to be processed, an inlet and an outlet for a processing liquid, and a hollow fiber membrane that isolates these liquids therebetween.
The hollow fiber membrane-based blood processing apparatus according to the present embodiment has: a container; a hollow fiber membrane bundle comprising a plurality of hollow fiber membranes, the bundle being inserted in this container; partitions wall by which both ends of this hollow fiber membrane bundle are respectively fixed to both ends of the container in a liquid-tight manner; processing liquid feed and exit ports communicated with a space in the container; and blood feed and exit ports respectively attached to both ends of the container and communicated with the bores of the hollow fiber membranes. Each of the hollow fiber membranes is the hollow fiber membrane for blood processing according to the preceding embodiment.
Figure 1 is a schematic cross-sectional view of the hollow fiber membrane-based blood processing apparatus according to the present embodiment.
A hollow fiber membrane-based blood processing apparatus 10 is loaded with a hollow fiber membrane bundle comprising a plurality of hollow fiber membranes 1 for blood processing, along the longitudinal direction of a tubular container 2.
Both ends of the hollow fiber membrane bundle are respectively fixed to both ends of the tubular container 2 by resins 3a and 3b (partition walls) such that the inside (first passages 1a) of the hollow fiber membranes 1 for blood processing is isolated from the outside (second passages 11) thereof. In this context, the second passages 11 include the space between the outside of the hollow fiber membranes 1 and the inner surface of the tubular container 2 as well as the space between a plurality of the hollow fiber membranes 1 for blood processing.
Each hollow fiber membrane 1 for blood processing has open ends, one of which allows a liquid to be processed such as blood to flow into the first passage 1a in a direction indicated by the arrow Fb through a space 8. The liquid to be processed such as blood that has passed through the first passage 1a can exit from the other open end. The tubular

container 2 are provided, at both ends, with header caps 7a and 7b with nozzles 6a and 6b, which face the end faces of the resins 3a and 3b, respectively, having the open ends of the hollow fiber membranes 1 on their surfaces and serve as feed and exit ports for the liquid to be processed such as blood.

Ports 2a and 2b which serve as feed and exit ports for a processing liquid such as a dialysis solution are disposed on the lateral faces of both ends of the tubular container 2. The processing liquid such as a dialysis solution can enter from, for example, the port 2b, in a direction indicated by the arrow Fa, then flow in the second passage 11, and exit from the port 2a. While flowing in the second passage 11, the processing liquid such as a dialysis solution can remove, for example, waste products from the liquid to be processed such as blood flowing in the first passage 1a via the hollow fiber membrane 1 for blood processing.

Examples of the hollow fiber membrane-based blood processing apparatus may include hemodialyzers, hemodialysis filters, hemofilters, continuous hemodialysis filters, continuous hemofilters, plasma separators, plasma fractionators, plasma component adsorbers, virus removers, blood concentrators, plasma concentrators, ascitic fluid filters, and ascitic fluid concentrators. Any medical device that exploits the filtration characteristics of the hollow fiber membrane is included in the blood processing apparatus according to the present invention.

(Polysulfone-based resin)

The polysulfone-based resin (hereinafter, also referred to as PSf) contained in the hollow fiber membrane for blood processing according to the present embodiment is a generic name for polymer compounds having a sulfone bond and is not particularly limited.

Examples thereof may include polysulfone polymers having repeat units represented by the formulas (1) to (5) shown below. In the formulas below, n is preferably an integer of 2 to 200, more preferably 80 to 140, further preferably 92 to 136.

The bisphenol-based polysulfone polymer of the formula (1) below is commercially available under the trade name of "Udel" from Solvay Advanced Polymers, LLC. or under the trade name of "Ultrason" from BASF Corp. and is found to be of several types, any of which may be used without particular limitations, depending on the degree of polymerization.

[Formula 1]

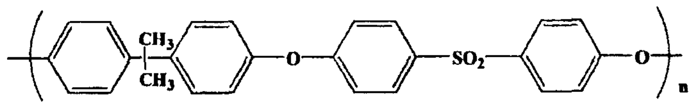

(1)

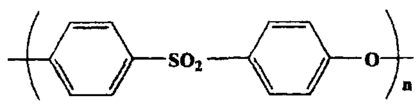

(2)

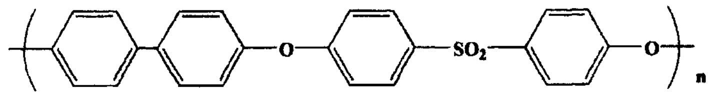

(3)

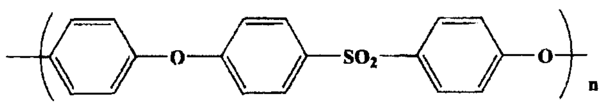

(4)

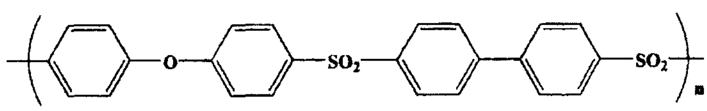

(5)

(Hydrophilic polymer)

The hydrophilic polymer contained in the hollow fiber membrane for blood processing according to the present embodiment refers to a polymer having a hydrophilic group.
Examples of the hydrophilic polymer may include, but not limited to, polyvinylpyrrolidone (hereinafter, also referred to as PVP), polyethylene glycol, polyvinyl alcohol, polypropylene glycol, and mixtures thereof. PVP is preferably used from the viewpoint of spinning stability and affinity for the polysulfone-based resin (PSf) described above.
PVP is found to be of several types depending on the degree of polymerization. For example, K-15, K-30, and K-90 differing in molecular weight are available under the trademark of "Plasdone" from ISP Ltd., and any of them may be used.

<Mass ratio of hydrophilic polymer to total mass of hydrophilic polymer and polysulfone-based resin in whole of hollow fiber membrane for blood processing (content A of hydrophilic polymer)>

The mass ratio of the hydrophilic polymer to the total mass of the hydrophilic polymer and the polysulfone-based resin in the whole of the hollow fiber membrane for blood processing according to the present embodiment (hereinafter, also referred to as a content A of the hydrophilic polymer) is 3% by mass or larger and 10% by mass or smaller.
The content A of the hydrophilic polymer is set to 3% by mass or larger in order to immobilize a practically sufficient amount of the hydrophilic polymer onto the hollow fiber membrane for blood processing as described later, and set to 10% by mass or smaller in order to obtain the practically sufficient tensile strength of the hollow fiber membrane for blood processing as well as its stable permeability in a harsh environment.
The content A of the hydrophilic polymer is preferably 4% by mass or larger and 9% by mass or smaller, more preferably 5% by mass or larger and 8% by mass or smaller.
Examples of a method for measuring the content A of the hydrophilic polymer may include a method using measurement results of 1H-NMR. Specifically, the method using 1H-NMR can involve: determining the molar ratio between the polysulfone-based resin and the hydrophilic polymer from the strength of peaks derived from the protons of a group specific for the polysulfone-based resin and the strength of peaks derived from the protons of a group specific for the hydrophilic polymer; and calculating the abundance ratio of the hydrophilic polymer in the whole of the hollow fiber membrane on the basis of the molar ratio.
For example, the polysulfone-based resin may have the structural unit represented by the formula (1), and the hydrophilic polymer may be polyvinylpyrrolidone. In such a case, focusing on the hydrogen atoms of one phenylene group in the structural unit of the polysulfone-based resin and hydrogen atoms in the structural unit of polyvinylpyrrolidone, the strength (integral values) of peaks attributed thereto is calculated for each of the compounds.
In 100 mol of the polysulfone-based resin, the phenylene group has four hydrogen atoms. Provided that the strength of peaks derived therefrom is 400, the strength of polyvinylpyrrolidone-derived peaks corresponds to the molar number of the polyvinylpyrrolidone with respect to 100 mol of the polysulfone resin.
On the basis of these results, the mass ratio between the compounds can be calculated. As a result, the abundance ratio of the hydrophilic polymer in the whole of the hollow fiber membrane is determined.

<Mass ratio of hydrophilic polymer to total mass of hydrophilic polymer and polysulfone-based resin in inner surface of hollow fiber membrane for blood processing (abundance ratio B of hydrophilic polymer)>

The mass ratio of the hydrophilic polymer to the total mass of the hydrophilic polymer and the polysulfone-based resin in the inner surface of the hollow fiber membrane for blood processing according to the present embodiment (hereinafter, also referred to as an abundance ratio B of the hydrophilic polymer) is 35% by mass or larger and 50% by mass or smaller.
The inner surface of the hollow fiber membrane for blood processing refers to the outermost surface on the inner side of the hollow fiber membrane, i.e., a hollow fiber membrane surface with which blood is contacted.
The abundance ratio B of the hydrophilic polymer can be set to 35% by mass or larger, thereby obtaining sufficient blood compatibility. The abundance ratio B of the hydrophilic polymer can be set to 50% by mass or smaller, thereby reducing the residual quantity of air after priming.
The abundance ratio B of the hydrophilic polymer is preferably 39% by mass or larger and 50% by mass or smaller, more preferably 40% by mass or larger and 50% by mass or smaller.
Examples of a method for measuring the abundance ratio B of the hydrophilic polymer may include a method using measurement results of X-ray photoelectron spectroscopy (XPS).
Specifically, the inner surface of the hollow fiber membrane is assayed by XPS to determine the ratio between the number of atoms specific for the polysulfone-based resin and the number of atoms specific for the hydrophilic polymer in this surface from the strength of peaks of these atoms. On the basis of this ratio, the mass ratio between these

compounds is obtained, and the abundance ratio can be calculated therefrom.

Specifically, in the case of using polyvinylpyrrolidone as the hydrophilic polymer, the abundance ratio of the polyvinylpyrrolidone is determined from the number of nitrogen atoms (derived from the polyvinylpyrrolidone) and the number of sulfur atoms (derived from the polysulfone-based resin) in the inner surface portion of the hollow fiber membrane.

For example, in the case of using the polysulfone-based resin having the structural unit represented by the formula (1) and polyvinylpyrrolidone as the hydrophilic polymer, the abundance ratio (% by mass) of the polyvinylpyrrolidone in the inner surface of the hollow fiber membrane can be determined according to the following expression (I):

[Expression 1]

$$\text{Abundance ratio [\%] of polyvinylpyrrolidone} = \frac{\text{The number of nitrogen atom} \times 111}{\text{The number of nitrogen atom} \times 111 + \text{The number of sulfur atom} \times 442} \times 100 \quad \cdots (I)$$

In the expression (I), 111 represents the formula weight of the repeat unit of polyvinylpyrrolidone; and 442 represents the formula weight of the repeat unit of polysulfone.

(Fat-soluble vitamin)

The fat-soluble vitamin contained in the hollow fiber membrane for blood processing according to the present embodiment generally refers to a vitamin that is poorly dissolved in water and is dissolved in alcohols or fats and oils.

Examples of the fat-soluble vitamin may include, but not limited to, vitamin A, vitamin D, vitamin E, vitamin K, and ubiquinone. Particularly, vitamin E is preferred.

Examples of the vitamin E may include, but not limited to, $\alpha$-tocopherol, $\alpha$-tocopherol acetate, $\alpha$-tocopherol nicotinate, $\beta$-tocopherol, $\gamma$-tocopherol, and $\delta$-tocopherol. Particularly, $\alpha$-tocopherol is preferably used because of its various physiological effects such as *in* vivo antioxidative effect, biomembrane stabilizing effect, and platelet aggregation inhibitory effect.

The fat-soluble vitamin plays a role in alleviating oxidative stress observed in patients receiving long-term dialysis, specifically, a role in eliminating peroxide causative of oxidative stress or allowing organisms to recover antioxidative effect.

The hollow fiber membrane for blood processing according to the present embodiment and the hollow fiber membrane-based blood processing apparatus provided with this hollow fiber membrane comprise the fat-soluble vitamin and the other components (hydrophilic polymer and polysulfone-based resin) in the hollow fiber membrane surface so as to each satisfy particular conditions described later, thereby producing effects such as the prevention of leak misdetection in a leak test as described later and improvement in the stability of permeability.

The hollow fiber membrane for blood processing according to the present embodiment seems to exert these effects by virtue of the fat-soluble vitamin present in the entire surface of the hollow fiber membrane. In this context, the "entire surface" includes the inner surface of the hollow fiber membrane with which blood is directly contacted as well as its outer surface and the inner surfaces of pores in a porous portion constituting the thickness of the membrane. Of blood components, blood cells come in contact only with the inner surface, whereas liquid components (e.g., proteins) or peroxides (e.g., active oxygen) are diffused to traverse the portion constituting the thickness of the membrane. Thus, the entire membrane surface from the porous portion to the outer surface contributes to antioxidative effect. In addition, a processing liquid (e.g., a wetting agent for leak inspection, a preservation solution, or a dialysis solution) or gas in the package bags of dry products comes in contact with the entire surface including the outer surface. In this respect, the total amount of fat-soluble vitamins present in the entire surface is of concern. In other words, fat-soluble vitamins buried in a membrane base material are excluded from the "abundance of the fat-soluble vitamin in the entire surface of the hollow fiber membrane" defined by the present embodiment, because these fat-soluble vitamins cannot come in contact with the liquid.

For the hollow fiber membrane for blood processing according to the present embodiment, the amount of the fat-soluble vitamin with respect to the mass of the hollow fiber membrane is consciously prescribed in order to define the abundance of the fat-soluble vitamin in the entire surface (including the porous portion constituting the thickness of the membrane, and the outer surface).

If the amount of the fat-soluble vitamin is defined with respect to the area (which generally refers to an inner surface area) of the membrane, the entire surface area largely differs depending on the difference in membrane thickness, albeit with a constant membrane area. As a result, the total amount of fat-soluble vitamins largely differs. For reliably obtaining predetermined effects, it is therefore difficult to prescribe the amount of the fat-soluble vitamin with respect to

the membrane area.

By contrast, the hollow fiber membrane for blood processing made of the polysulfone-based resin, as in the present embodiment, has an almost constant porosity even if the membrane thickness varies. Thus, the range of the abundance that produces excellent effects can be prescribed with respect to the volume or the mass.

Thus, placing much value on measurement reproducibility for the hollow fiber membrane for blood processing according to the present embodiment, the abundance of the fat-soluble vitamin in the entire surface of the hollow fiber membrane for blood processing is prescribed with respect to the mass of the hollow fiber membrane for blood processing.

In the hollow fiber membrane for blood processing according to the present embodiment, the abundance of the fat-soluble vitamin in the entire surface of the hollow fiber membrane for blood processing is 0.5 mg or larger and 25 mg or smaller per g of the hollow fiber membrane.

The abundance of the fat-soluble vitamin in the entire surface of the hollow fiber membrane for blood processing can be set to 0.5 mg or larger and 25 mg or smaller based on 1 g of the hollow fiber membrane, thereby obtaining excellent blood compatibility.

The abundance of the fat-soluble vitamin in the entire surface of the hollow fiber membrane for blood processing is preferably 1.5 mg or larger and 18 mg or smaller, more preferably 1.5 mg or larger and 15 mg or smaller, based on 1 g of the hollow fiber membrane.

In the present specification, this amount is also referred to as an "abundance of the fat-soluble vitamin in the entire surface of the hollow fiber membrane".

<Method for measuring abundance of fat-soluble vitamin in entire surface of hollow fiber membrane>

The abundance of the fat-soluble vitamin in the entire surface of the hollow fiber membrane for blood processing according to the present embodiment can be evaluated by injecting an aqueous alcohol solution, an aqueous surfactant solution, or the like to the hollow interior of the hollow fiber membrane-based blood processing apparatus to extract the fat-soluble vitamin in the inner surface of the hollow fiber membrane, followed by quantification by liquid chromatography.

In this procedure, all fat-soluble vitamins present on the entire surface of the hollow fiber membrane for blood processing must be extracted. Thus, the extraction solvent needs to be highly capable of dissolving fat-soluble vitamins. Meanwhile, if a membrane base material is swollen, even fat-soluble vitamins buried in the membrane base material may be extracted. For these reasons, an aqueous surfactant solution incapable of swelling the membrane base material and capable of sufficiently dissolving fat-soluble vitamins is desirably used as the extraction solvent.

A specific example of the method for measuring the abundance of the fat-soluble vitamin present in the entire surface of the hollow fiber membrane for blood processing will be shown below.

In this context, the method for measuring the abundance of the fat-soluble vitamin present in the entire surface of the hollow fiber membrane for blood processing is not limited to the example shown below, and the amount of samples, the concentration or amount of an extracting solution, temperatures, times, flow rates of liquid to be injected, measurement apparatuses, etc. can be appropriately adjusted within ranges that satisfy the purposes described above.

First, the hollow fiber membrane-based blood processing apparatus is disassembled to collect the hollow fiber membranes for blood processing, which are in turn rinsed and then dried.

Subsequently, 4 g of the hollow fiber membranes for blood processing thus dried is weighed into a vial. For example, 80 mL of an aqueous solution containing 1% by mass of polyethylene glycol-t-octyl ether is added thereto as an aqueous surfactant solution. The fat-soluble vitamin is extracted at room temperature for 60 minutes under ultrasonic vibration. The fat-soluble vitamin is quantified by liquid chromatography. The amount of the fat-soluble vitamin in the extract is determined using a calibration curve obtained from the peak area of a standard fat-soluble vitamin solution. An example of the quantification method by liquid chromatography is as follows: a column (Shodex Asahipak ODP-506E packed column for HPLC) is attached to a high-performance liquid chromatography apparatus (pump: PU-1580 manufactured by JASCO Corp., detector: RID-6A manufactured by Shimadzu Corp., autoinjector: SIL-6B manufactured by Shimadzu Corp., data processing: GPC-8020 manufactured by Tosoh Corp., column oven: 556 manufactured by GL Sciences Inc.). A mobile phase methanol for high-performance liquid chromatography is applied to the column (temperature: 40°C), for example, at a flow rate of 1 mL/min. The fat-soluble vitamin concentration is determined from the area of an absorption peak in the ultraviolet region. From this concentration, the mass (mg/g) of the entire-surface fat-soluble vitamins contained in the hollow fiber membrane for blood processing is determined with extraction efficiency as 100%. Some fat-soluble vitamins are inactivated by exposure to radiation. The abundance of the fat-soluble vitamin in the entire surface of the hollow fiber membrane for blood processing constituting the hollow fiber membrane-based blood processing apparatus according to the present embodiment also includes the amount of such fat-soluble vitamins inactivated by exposure to radiation.

[Characteristics of hollow fiber membrane for blood processing]

(Blood compatibility of hollow fiber membrane constituting hollow fiber membrane-based blood processing apparatus)

Heretofore, gradual improvement in blood compatibility has been pursued for hollow fiber membrane-based blood processing apparatuses. Nonetheless, the blood compatibility is still susceptible to improvement owing to, for example, inevitable combined use with an anticoagulant or various intercurrent complications resulting from long-term repeated use.

As in conventional techniques, a fat-soluble vitamin-free polysulfone-based hollow fiber membrane containing a hydrophilic polymer was covered with an aqueous glycerin solution exemplified as a "wet protectant" and further sterilized with electron beam. The resulting membrane was evaluated for its blood compatibility by [Determination of lactate dehydrogenase (LDH) activity] shown later in Examples. As a result, the blood compatibility was confirmed to be still susceptible to improvement (see Patent Document 3 (Japanese Patent Laid-Open No. 2008-93228); which will be described later in detail in Comparative Example 10).

In the description below, a level at which blood compatibility can be found to be "susceptible to improvement" under a more strict testing method newly developed by the present inventors is expressed as "insufficient". However, this expression is used to intend a higher level of blood compatibility.

Patent Document 3 described above states that an aqueous polyhydric alcohol solution has the ability to scavenge radical species formed during radiation sterilization and therefore prevents the membrane-surface hydrophilic polymer responsible for blood compatibility from being degraded (depolymerized or excessively crosslinked) by the radical species.

The insufficient blood compatibility, however, may suggest that the aqueous glycerin solution was lacking in the ability to scavenge a large amount of radical species formed during radiation sterilization.

In order to seek the higher ability to scavenge radical species, vitamin E also exemplified in Patent Document 3 was added to the hollow fiber membrane so as not to clog fine pores in the hollow fiber membrane surface, and the resulting membrane was further sterilized with electron beam. As a result, sufficient blood compatibility was not obtained yet.

The amount of vitamin E attached to the membrane was a level lower than 60 to 400% required for the "wet protectant" in Patent Document 3 and however, secured a sufficient amount of vitamin E remaining in the hollow fiber membrane even after sterilization through the reaction of an aqueous ferric chloride solution described later. The resulting membrane surface had the sufficient ability to scavenge radical species. Thus, it seems unlikely that a lack of the ability to scavenge radical species was responsible for the insufficient blood compatibility.

The present inventors further considered that reduction in blood compatibility presumably caused by the modification of the hydrophilic polymer could be compensated for by increase in the abundance of the hydrophilic polymer, and conducted the following experiment:

Firstly, the polyvinylpyrrolidone (PVP; hydrophilic polymer) concentration of a dope for formation of the hollow fiber membrane was raised to increase the PVP concentration of the inner surface of the hollow fiber membrane. The obtained hollow fiber membrane-based blood processing apparatus of dry type still had insufficient blood compatibility and had a larger amount of effluents from the hollow fiber membrane. In addition, relative decrease in PSf composition in the hollow fiber membrane base material reduced hollow fiber membrane strength. Thus, the resulting hollow fiber membrane was unstably resistant to a leak in use (which will be described later in detail in Comparative Example 6).

Secondly, an aqueous PVP solution was injected to a fat-soluble vitamin-free hollow fiber membrane-based blood processing apparatus, which was then dried and radiation-sterilized. The apparatus obtained by this method had satisfactory blood compatibility by the addition of an excess of PVP, but was unable to be used as a blood processing apparatus because air was not completely removed even by priming (operation of wetting the membrane by the injection of water while removing gas in the hollow interior) before use (which will be described later in detail in Comparative Example 7).

Thus, a hollow fiber membrane-based blood processing apparatus that satisfied the blood compatibility and priming performance of interest was not obtained easily using the conventional techniques themselves or with creative ingenuity.

As a result of conducting further studies, the present inventors found that, surprisingly, a radiation-sterilized hollow fiber membrane-based blood processing apparatus provided with a fat-soluble vitamin-containing hollow fiber membrane covered on its inner surface with a hydrophilic polymer in an amount much smaller than that required for a fat-soluble vitamin-free hollow fiber membrane to secure blood compatibility has favorable blood compatibility and priming performance as well as sufficient membrane strength.

In the hollow fiber membrane-based blood processing apparatus and the hollow fiber membrane for blood processing according to the present embodiment, the abundance of the fat-soluble vitamin in the entire surface of the hollow fiber membrane for blood processing is, as described above, 0.5 mg or larger and 25 mg or smaller based on 1 g of the hollow fiber membrane.

Under this condition, the content A of the hydrophilic polymer is set to 3% by mass or larger and 10% by mass or smaller, and the abundance ratio B of the hydrophilic polymer is set to 35% by mass or larger and 50% by mass or smaller.

Provided that the abundance of the fat-soluble vitamin in the entire surface of the hollow fiber membrane is 0.5 mg or larger per g of the hollow fiber membrane, the hydrophilic polymer can be sufficiently distributed on the blood-contact side, thereby obtaining favorable blood compatibility.

Accordingly, the abundance of the fat-soluble vitamin in the entire surface of the hollow fiber membrane is 0.5 mg or larger, preferably 1.0 mg or larger, more preferably 1.5 mg or larger.

Alternatively, the abundance of the fat-soluble vitamin in the entire surface of the hollow fiber membrane can be set to 25 mg or smaller based on 1 g of the hollow fiber membrane, thereby suppressing the influence of hydrophobicity of the fat-soluble vitamin itself. The resulting hollow fiber membrane can produce practically sufficient antithrombotic properties and can prevent blood from coagulating in the hollow fiber upon contact of the membrane with blood, i.e., can prevent so-called residual blood.

Accordingly, the upper limit of the abundance of the fat-soluble vitamin in the entire surface of the hollow fiber membrane is 25 mg or smaller, preferably 18 mg or smaller, more preferably 15 mg or smaller.

The hollow fiber membrane for blood processing according to the present embodiment containing a particular amount of a fat-soluble vitamin as described above, which constitutes the hollow fiber membrane-based blood processing apparatus according to the present embodiment, exhibits high blood compatibility by the covering of its inner surface with a particular amount of a hydrophilic polymer as described above and further radiation sterilization. This mechanism is probably as follows:

As shown in Figure 2, a polysulfone/hydrophilic polymer blend membrane has a surface in the state where hydrophilic polymers 42 exist to protrude from a membrane base material 41 made of a hydrophobic polysulfone polymer.

The hydrophilic polymers 42 are hydrated in blood to form a diffuse layer indispensable for retaining blood compatibility. This diffuse layer of the hydrophilic polymers 42 has a thickness 43.

A hollow fiber membrane-based blood processing apparatus of wet type in which the whole of the hollow fiber membrane is filled with water is known to substantially maintain the state of Figure 2 even after radiation sterilization resulting in the slight modification of hydrophilic polymers. Specifically, this apparatus sufficiently secures the thickness 43 of hydrophilic polymers that are hydrated in blood to form a diffuse layer indispensable for retaining blood compatibility.

Figure 3 is a model diagram showing the state of a hollow fiber membrane having dry surface in a radiation-sterilized hollow fiber membrane-based blood processing apparatus of dry type.

In Figure 3, hydrophilic polymers 51 are modified (e.g., depolymerized) by the action of radical species or the like formed by radiation sterilization.

As a result, the hydrophilic polymers 51 that form a diffuse layer in blood has a thickness 52, which is smaller than that of the hydrophilic polymers 42 shown in Figure 2. The resulting membrane fails to exhibit sufficient blood compatibility.

Figure 4 is a model diagram showing the surface state of a hollow fiber membrane obtained by further adding hydrophilic polymers in a solution form to the polysulfone/hydrophilic polymer blend membrane having the protruding hydrophilic polymers 42 shown in Figure 2, and removing the solvent by drying to immobilize the additional hydrophilic polymers thereon.

Additional hydrophilic polymers 62 are brought into contact with the membrane base material 41, as in the hydrophilic polymers 42 residing from membrane formation. In this state, the membrane is radiation-sterilized. Figure 5 is a model diagram showing the surface state of the resulting membrane.

The originally residing hydrophilic polymers 42 and the additional hydrophilic polymers 62 are equally modified (e.g., depolymerized) into hydrophilic polymers 71 and additional hydrophilic polymers 72, respectively. As a result, the diffuse layer has a smaller thickness 73 in blood. The resulting membrane fails to exhibit sufficient blood compatibility.

Figure 6 is a model diagram showing the surface state of a hollow fiber membrane obtained by adding a solution containing fat-soluble vitamins and hydrophilic polymers to the polysulfone/hydrophilic polymer blend membrane having the protruding hydrophilic polymers 42 shown in Figure 2, and then removing the solvent by drying to immobilize the fat-soluble vitamins and the additional hydrophilic polymers thereon.

An aqueous alcohol solution is preferably used as a solvent that co-dissolves the fat-soluble vitamins and the hydrophilic polymers and has no influence on the hollow fiber membrane base material and the originally residing hydrophilic polymers 42.

The alcohol first volatilizes in the solvent drying process so that fat-soluble vitamins 81 are separated to cover the strongly hydrophobic polysulfone base material surface 41.

As a result, additional hydrophilic polymers 83, which repulse the strongly hydrophobic fat-soluble vitamins 81, are moved toward a side opposite to the fat-soluble vitamin 81-attached membrane surface side on the originally residing hydrophilic polymers 42, i.e., toward the blood-contact side of the hollow fiber membrane-based blood processing apparatus to be finally obtained, and immobilized thereon by the removal of water by drying.

In this context, the originally residing hydrophilic polymers 42 are probably elongated toward the blood-contact side through repulsion for the fat-soluble vitamins 81.

In this state, the membrane is radiation-sterilized. Figure 7 is a model diagram showing the surface state of the resulting membrane.

The originally residing hydrophilic polymers 42 and the additional hydrophilic polymers 83 are equally modified (e.g., depolymerized) into hydrophilic polymers 91 and 92, respectively. Since the additional hydrophilic polymers 92 are positioned on the blood-contact side, the resulting diffuse layer in blood has a thickness 93, which is substantially equivalent to that in the hollow fiber membrane-based blood processing apparatus of wet type. Thus, the resulting membrane can exhibit sufficient blood compatibility.

In addition, this approach can enhance wettability at the initial stage after the start of use and is consequently effective in improving affinity for blood.

The additional hydrophilic polymers 83 are immobilized on the membrane by intertwining with the originally residing hydrophilic polymers 42.

In this respect, this immobilization is not sufficiently performed unless the originally residing hydrophilic polymers 42 are present in a certain amount or more. In this case, the effects of the present invention cannot be obtained. For this reason, the content A of the hydrophilic polymer, which reflects the amount of the originally residing hydrophilic polymers 42, in the whole of the hollow fiber membrane needs to be 3% by mass or larger. As a result, the hydrophilic polymers are sufficiently immobilized thereon. On the other hand, the upper limit of the content A is 10% by mass or smaller. The content A can be set to 10% by mass or smaller, thereby obtaining the practically sufficient tensile strength and toughness of the hollow fiber membrane. The toughness will be described later.

The presence of the hydrophilic polymer in the inner surface of the hollow fiber membrane is essential for enhancing blood compatibility.

Various parameters are known for blood compatibility. The blood compatibility may be represented by a measurement value of lactate dehydrogenase (LDH) activity described in detail in Examples.

This index is an index substituted for the amount of platelet attached. In actual clinical application, however, its desirable measurement value differs depending on the anticoagulant (e.g., heparin) used. In fact, even a hollow fiber membrane having a measurement value of 300 points is clinically used. For the purpose of the present invention aimed at a higher level of blood compatibility, the goal is to achieve measurement value of 100 or lower. A measurement value of 50 or lower can achieve a much higher level of blood compatibility. A measurement value of 10 or lower is further preferred.

The abundance ratio B of the hydrophilic polymer, which is the mass ratio of the hydrophilic polymer to the total mass of the polysulfone-based resin and the hydrophilic polymer in the inner surface of the hollow fiber membrane for blood processing according to the present embodiment, is 35% by mass or larger and 50% by mass or smaller. Provided that this abundance ratio B is 35% by mass or larger, practically sufficient blood compatibility can be obtained. The abundance ratio B is preferably 39% by mass or larger, more preferably 40% by mass or larger.

On the other hand, the upper limit of the abundance ratio B is 50% by mass. The abundance ratio B can be set to 50% by mass or smaller, thereby sufficiently reducing the residual quantity of air after priming of the hollow fiber membrane-based blood processing apparatus.

A residual quantity of air on the order of a few mL is not problematic for practical use. By contrast, a residual quantity of air exceeding 5 mL poses a risk of sending air into patient's body beyond a blood chamber. Thus, the abundance ratio B of the hydrophilic polymer in the inner surface of the hollow fiber membrane is 35% by mass or larger and 50% by mass or smaller, preferably 39% by mass or larger and 50% by mass or smaller, more preferably 40% by mass or larger and 50% by mass or smaller. A specific method for evaluating the residual quantity of air will be described in detail in Examples.

(Leak characteristics of hollow fiber membrane-based blood processing apparatus)

Examples of a leak test method that can be applied to the evaluation of the leak characteristics of the hollow fiber membrane-based blood processing apparatus according to the present embodiment may include a method comprising a step of coating the hollow fiber membrane with a liquid harmless to organisms, such as water, and a leak test step of determining the gas permeability of the hollow fiber membrane thus coated.

Hereinafter, this leak test method will be described with reference to each step.

<Step of coating hollow fiber membrane>

The liquid for coating the hollow fiber membrane is preferably, a hydrophilic liquid (coating solution), for example, water or an aqueous solution, which dissolves neither the hollow fiber membrane nor the fat-soluble vitamin, is harmless to organisms, and offers the high wettability of the hollow fiber membrane. Such a liquid is removed by the drying of the hollow fiber membrane after the leak test. The liquid, even if left to some extent on the membrane, can be easily removed by the washing of the hollow fiber membrane with a saline solution or the like before use.

The hollow fiber membrane may be coated, together with a dense layer formed in the vicinity of the inner surface of the hollow fiber membrane, with the coating solution described above. In this case, for example, a method which involves injecting the coating solution to the inside (first passages 1a) of the hollow fiber membranes 1 constituting the hollow fiber membrane-based blood processing apparatus shown in Figure 1 can be adopted as a coating method. By this method, the coating solution comes in contact with at least the inner surface of each hollow fiber membrane 1. The redundant solution can be removed by flash (blow-off) using gas or the like or by draining under centrifugal force. Such a method can impregnate the hollow fiber membrane with the coating solution, thereby drastically reducing the gas permeability of the hollow fiber membrane. In this state, the presence or absence of a pinhole can be easily determined in the leak test step described later.

<Leak test step of determining gas permeability>

The hollow fiber membrane thus coated with the coating solution, together with a dense layer formed in the vicinity of the inner surface of the hollow fiber membrane, is subjected to a leak test.

In the leak test, the presence or absence of a pinhole in the hollow fiber membrane is determined.

Examples of a method therefor may include a method which involves pressurizing the hollow fiber membrane with gas, measuring the rate at which the gas penetrates the hollow fiber membrane, and determining the presence or absence of a pinhole on the basis of the rate.

More specifically, a given air pressure is applied to the inside (first passages 1a) of the hollow fiber membranes 1 in the hollow fiber membrane-based blood processing apparatus shown in Figure 1. Then, the pressurization is stopped, and the openings are closed. Pressure drop within the hollow fiber membranes 1 is measured to examine the presence or absence of a pinhole. When the pressure drop occurs at a level exceeding the normal level, the pinhole is present. Thus, a leak can be determined to occur.

A conventional highly gas-permeable hollow fiber membrane known in the art, however, may cause leak misdetection due to pressure drop resulting from the insufficiently coated inner surface of the hollow fiber membrane, even if coated by the method described above. Specifically, water supposed to clog fine pores may fail to form a water film because it passes through the fine pores by the pressure of air flash for the purpose of uniformly forming a coating layer. Alternatively, fine pores may be too large to resist gas pressure for leak inspection. Thus, the water film cannot be maintained. These factors may lead to pressure drop.

For these reasons, an accurate leak test is difficult to conduct on the conventional highly gas-permeable hollow fiber membrane known in the art.

By contrast, according to the hollow fiber membrane for blood processing of the present embodiment, the abundance of the fat-soluble vitamin in the entire surface of the hollow fiber membrane is 0.5 mg or larger and 25 mg or smaller based on 1 g of the hollow fiber membrane, and the abundance ratio B of the hydrophilic polymer, which is the mass ratio of the hydrophilic polymer to the total mass of the polysulfone-based resin and the hydrophilic polymer in the inner surface of the hollow fiber membrane, is 35% by mass or larger and 50% by mass or smaller, whereby the inner surface of the hollow fiber membrane is rendered excellently wettable with the coating solution and the gas permeability of the hollow fiber membrane is sufficiently reduced. As a result, leak misdetection can be reduced.

Since the fat-soluble vitamin is highly hydrophobic, the presence of the fat-soluble vitamin in hollow fiber membrane surface is generally predicted to rather reduce wettability and increase the rate of leak misdetection. Thus, the fat-soluble vitamin is usually considered to be ineffective for reducing leak misdetection as described above. By contrast, the hollow fiber membrane for blood processing according to the present embodiment and the hollow fiber membrane-based blood processing apparatus provided with this hollow fiber membrane produce the effect of sufficiently reducing the gas permeability of the inner surface of the hollow fiber membrane. A reason for this is presumably as described below.

Figure 8 is a model diagram showing a region in the vicinity of the surface of a hollow fiber membrane consisting only of a base material of a polysulfone-based resin 151 and hydrophilic polymers 152.

The polysulfone-based resin 151 originally has a highly hydrophobic surface. Upon wetting with water, however, the surface is rendered easily wettable in the presence of the hydrated hydrophilic polymers 152. As a result, a water film that clogs fine pores can be formed on the surface.

In this case, the hydrophilic polymers 152 exert their maximum effects in a sufficiently elongated state, but

cannot sufficiently retain the water film if the base material of the polysulfone-based resin 151 has a large pore size and high permeability.

By contrast, the hollow fiber membrane for blood processing according to the present embodiment has elongated hydrophilic polymers 42 and a highly dense state attributed to the later-added hydrophilic polymers 83, as described with reference to Figure 6. This brings about enhancement in the water wettability of the hollow fiber membrane surface and reduction in the substantial pore size of fine pores.

As described above, the coexistence of the fat-soluble vitamin and the hydrophilic polymer in certain amounts or more achieves the rate of leak misdetection low enough for practical use.

The model described above is of a hollow fiber membrane wherein the abundance of the fat-soluble vitamin in the entire surface of the hollow fiber membrane falls within the preferred range (0.5 mg or larger and 25 mg or smaller per g of the hollow fiber membrane).

By contrast, the fat-soluble vitamin may be present in excessive abundance to displace the hydrophilic polymer so that the fat-soluble vitamin is exposed at the surface of fine pores. In such a case, wettability is sharply reduced due to an upsurge in the hydrophobicity of the hollow fiber membrane surface. This results in sharp increase in the rate of leak misdetection.

As for the upper limit of the abundance of the fat-soluble vitamin in the hollow fiber membrane for blood processing according to the present embodiment, a preferred value determined from the viewpoint of the rate of leak misdetection described above is almost the same as that determined from the viewpoint of blood compatibility. This is because the amount of the fat-soluble vitamin can be controlled to a level that renders the fat-soluble vitamin sufficiently maskable by the originally residing hydrophilic polymers and the later-added hydrophilic polymers, thereby reliably securing the easy formation of a water film on the inner surface of the hollow fiber membrane and achieving the easy adsorption of proteins and blood cells.

Meanwhile, it is difficult to obtain sufficiently consistent leak test results by merely prescribing the abundance ratio B of the hydrophilic polymer in the inner surface of the hollow fiber membrane and the abundance of the fat-soluble vitamin in the entire surface of the hollow fiber membrane.

This is presumably because the hollow fiber membrane surface may be structurally changed if the rate of contraction of the hollow fiber membrane varies due to the biased abundance ratio of the hydrophilic polymer in the whole of the hollow fiber membrane.

Thus, the content A of the hydrophilic polymer, which is the mass ratio of the hydrophilic polymer to the total mass of the hydrophilic polymer and the polysulfone-based resin in the inner surface of the hollow fiber membrane and the whole of the hollow fiber membrane, is adjusted to 3% by mass or larger and 10% by mass or smaller. In addition, the ratio of the abundance ratio B of the hydrophilic polymer in the inner surface of the hollow fiber membrane to the content A of the hydrophilic polymer in the whole of the hollow fiber membrane is set to 8.0 or larger and 50 or smaller. As a result, the leak test has been found to produce exceedingly consistent results.

These conditions can be satisfied in combination in addition to the condition of the abundance ratio B of the hydrophilic polymer in the inner surface of the hollow fiber membrane, thereby producing highly reproducible results with far less leak misdetection. When these conditions are satisfied, the state of the inner surface of the hollow fiber membrane (or the dense layer on the hollow fiber membrane) (specifically, uneven states where the polysulfone-based resin and the hydrophilic polymer are present, uneven pore sizes, uneven thicknesses, etc.) is presumably rendered easily maskable by the coating solution.

The degree to which leak misdetection occurs can be evaluated on the basis of the incidence of leak misdetection (rate of leak misdetection). The rate of leak misdetection can be calculated, for example, by the following method:

> Specifically, a plurality of hollow fiber membrane-based blood processing apparatuses are subjected to the leak test method described above. Of them, the number of apparatuses confirmed to have a pinhole is calculated. Then, the apparatuses confirmed to have a pinhole are subjected again to the leak test method. In this case, an apparatus confirmed to have a pinhole in the first test but confirmed to have no pinhole in the second test corresponds to leak misdetection. Thus, the rate of leak misdetection is defined as the ratio of the number of samples with leak misdetection to the number of samples confirmed to have a pinhole in the first test. A larger value of this ratio means a higher incidence of leak misdetection.

(Stable permeability of hollow fiber membrane in harsh environment)

Stabile permeability in a harsh environment, i.e., stability against degradation in permeability caused by storage in a harsh environment (hereinafter, also simply referred to as stability), is influenced by the abundance ratio B of the hydrophilic polymer in the inner surface of the hollow fiber membrane and the abundance of the fat-soluble vitamin in the entire surface of the hollow fiber membrane.

Specifically, the abundance of the fat-soluble vitamin in the entire surface of the hollow fiber membrane is set

to 0.5 mg or larger and 25 mg or smaller based on 1 g of the hollow fiber membrane. In this case, the stability is reduced if the abundance ratio B of the hydrophilic polymer is smaller than 35% by mass.

Storage in a harsh environment causes the oxidative degradation (depolymerization) of the hydrophilic polymer, which in turn changes permeability, specifically, excessively decreases or increases permeability. From a commonsense perspective, the fraction of the hydrophilic polymer should be decreased in order to enhance the stability. Improvement in the stability, however, requires that, as described above, the abundance of the fat-soluble vitamin in the entire surface of the hollow fiber membrane should be set to 0.5 mg or larger and 25 mg or smaller per g of the hollow fiber membrane and in addition, the hydrophilic polymer should be 35% by mass or larger. Even in the presence of the fat-soluble vitamin, some hydrophilic polymers are oxidatively degraded. The initial abundance of the hydrophilic polymer must therefore be a certain amount or more.

The presence of the fat-soluble vitamin further suppresses the oxidative degradation of the hydrophilic polymer caused by long-term storage in a high-temperature atmosphere and consequently also contributes to the prevention of change in water permeability.

In addition, the content A of the hydrophilic polymer also influences the stability. The backbone of the hollow fiber membrane according to the present embodiment is constituted by a complex of the polysulfone-based resin and the hydrophilic polymer. Unlike the polysulfone-based resin, which is highly heat-resistant, the hydrophilic polymer that has absorbed water is low resistant to heat. For this reason, the structure of a membrane having a higher content A may be deformed in a harsh environment to degrade permeability. In order to prevent this, the content A is set to 10% by mass or smaller.

In this context, the stability of water permeability can be determined on the basis of the rate of change in the amount of water permeating the blood processing apparatus, specifically, the rate of change in ultrafiltration rate (UFR), between before and after a model test involving heating for a given period. The membrane can be regarded as having excellent stability when this rate of change in ultrafiltration rate is within ±10%. A rate of change exceeding this range might cause clinically unacceptable change in solute removal performance. From this point of view, the hollow fiber membrane having excellent stability can maintain the desired permeability even after long-term storage in a high-temperature atmosphere.

Accordingly, the abundance of the fat-soluble vitamin in the entire surface of the hollow fiber membrane needs to be 0.5 mg or larger based on 1 g of the hollow fiber membrane for the temporal stability of the permeability of the hollow fiber membrane and the stability of permeability against storage in a harsh environment. The abundance is preferably 1.0 mg or larger, more preferably 1.5 mg or larger. In addition, the abundance ratio B of the hydrophilic polymer is set to 35% by mass or larger.

(Residual quantity of air after priming)

Before use of the hollow fiber membrane-based blood processing apparatus in a dry state accommodating the hollow fiber membrane for blood processing, saline or the like is generally injected into the apparatus to render the membrane surface wet. This priming operation might encounter the trouble of failing to expel air from the hollow portion.

An amount of remaining air, i.e., a residual quantity of air, on the order of a few mL is not problematic for practical use. By contrast, a residual quantity of air exceeding 5 mL poses a risk of sending air into patient's body beyond a blood chamber. This is due to the poor water wettability of the hollow fiber membrane. From a commonsense perspective, the abundance ratio B of the hydrophilic polymer in the inner surface of the hollow fiber membrane may be increased, thereby producing the effect of improving water wettability.

The hollow fiber membrane according to the present embodiment containing the fat-soluble vitamin, however, exhibits an upsurge in the residual quantity of air after priming if the abundance ratio B of the hydrophilic polymer in the inner surface of the hollow fiber membrane exceeds 50% by mass. A reason for this is uncertain, but is probably that: a film of excessive hydrophilic polymers clogs the hollow portion of the hollow fiber membrane and a priming liquid no longer flows in the clogged hollow fiber membrane at a low pressure or at a low flow rate, resulting in an air pocket.

Thus, the abundance ratio of the hydrophilic polymer in the inner surface of the hollow fiber membrane is 35% by mass or larger and 50% by mass or smaller, preferably 39% by mass or larger and 50% by mass or smaller, more preferably 40% by mass or larger and 50% by mass or smaller. A specific method for evaluating the residual quantity of air will be described in detail in Examples.

(Elution from hollow fiber membrane)

Elution from the hollow fiber membrane into blood during use of the membrane might adversely affect the organism to which the hollow fiber membrane is applied.

Such elution into blood, which is an aqueous medium, is mostly derived from the amount of the hydrophilic polymer. For this reason, the content A of the hydrophilic polymer in the whole of the hollow fiber membrane and the

abundance ratio B of the hydrophilic polymer in the inner surface must be minimized.

Nonetheless, particularly, too low a content A of the hydrophilic polymer inhibits the immobilization of hydrophilic polymers added later by coating. Thus, the content A of the hydrophilic polymer needs to be a certain amount or more.

In addition, the hydrophilic polymer is cross-linked by exposure to radiation during sterilization and thus prevented from being eluted from the hollow fiber membrane into blood. On the other hand, the hydrophilic polymer may be depolymerized by the radiation or radical species formed by the radiation so that its elution is promoted.

Mixing with the fat-soluble vitamin is effective for suppressing such radiation depolymerization of the hydrophilic polymer. Thus, this fat-soluble vitamin needs to be used in a proper amount.

From these points of view, the abundance of the fat-soluble vitamin in the entire surface of the hollow fiber membrane is set to 0.5 mg or larger and 25 mg or smaller based on 1 g of the membrane, the content A of the hydrophilic polymer in the whole of the hollow fiber membrane is set to 3% by mass or larger and 10% by mass or smaller, and the abundance ratio B of the hydrophilic polymer in the inner surface of the hollow fiber membrane is set to 50% by mass or smaller.

The elution from the hollow fiber membrane can be evaluated, for example, by the following method.

Specifically, the hollow fiber membrane-based blood processing apparatus after priming is disassembled to recover the hollow fiber membranes, which are in turn dipped in pure water of 70°C for 1-hour extraction. The absorbance of the extract that exhibits the maximum absorption in the UV spectrum at 350 to 220 nm can be used as an index substituted for the amount of effluents.

(Toughness of hollow fiber membrane)

The hollow fiber membrane is housed in a container and used in a module form. A membrane having insufficient mechanical strength carries a risk of being broken during module production or handling.

The mechanical strength can be represented by toughness obtained according to a tensile test. Specifically, each hollow fiber membrane having toughness of 1000 gf.% is practically sufficient.

The toughness in the present specification refers to a value determined according to the expression Stress at break (gf) x Degree of elongation (%). A method for measuring the toughness will be described in detail in analytical methods of Examples.

The polysulfone resin (PSf) is responsible for the strength of the hollow fiber membrane. From the viewpoint of strength, a large content of the hydrophilic polymer coexisting therewith is not preferred. The excessive fraction of the hydrophilic polymer causes sharp decrease in membrane strength. The content A of the hydrophilic polymer in the whole of the hollow fiber membrane therefore needs to be 10% by mass or smaller and is preferably 9% by mass or smaller, more preferably 8% by mass or smaller.

(Content of nitric acid ion in hollow fiber membrane for blood processing)

Preferably, the hollow fiber membrane for blood processing according to the present embodiment contains 1 ppm or higher and 8 ppm or lower nitric acid ions.

In the case of using a nitrogen atom-containing polymer (e.g., polyvinylpyrrolidone) as the hydrophilic polymer, nitric acid ions are generated in the separation membrane by radiation sterilization in a dry state. The resulting hollow fiber membrane for blood processing contains a nitric acid ion component.

It is obvious that a membrane that has a larger amount of the nitrogen atom-containing polymer or receives a larger dose of radiation has a larger content of nitric acid ions.

The abundance of nitric acid ions in the hollow fiber membrane for blood processing according to the present embodiment, however, is controlled to a relatively low level with high reproducibility, as compared with a fat-soluble vitamin-free membrane, by virtue of the moderate antioxidative effect of the fat-soluble vitamin and further the appropriate arrangement of the hydrophilic polymer and the fat-soluble vitamin shown in Figure 6 at a radiation sterilization dose ranging from 15 to 50 kGy, which is general for blood processing membranes.

In the hollow fiber membrane for blood processing according to the present embodiment, the nitric acid ions probably exist in the form of nitric acid.

An excess of nitric acid is not preferred because of promoting the alteration of the hollow fiber membrane for blood processing due to oxidative effect or the like.

By contrast, the presence of nitric acid in a moderate amount improves blood compatibility (LDH activity in Examples) and improves the ease of priming (residual quantity of air in Examples). This is probably because the presence of nitric acid increases the hydroscopic properties of the hydrophilic polymer to improve its hydrophilicity, and on the other hand, suppresses the dissociation of a phenolic hydroxy group from the fat-soluble vitamin to improve its hydrophobicity.

As a result, the hydrophilic-hydrophobic repulsion between the hydrophilic polymer and the fat-soluble vitamin,

as shown in Figure 6, is enhanced. The resulting hollow fiber membrane for blood processing is rendered more water-wettable and exhibits improved blood compatibility and ease of priming.

The range of the nitric acid ion component which is effective for increasing the hydroscopic properties of the hydrophilic polymer to improve its hydrophilicity and suppressing the dissociation of a phenolic hydroxy group from the fat-soluble vitamin to improve its hydrophobicity, as described above, and has no adverse effect, is preferably 1 ppm or higher and 8 ppm or lower, more preferably 2 ppm or higher and 6 ppm or lower, in terms of its concentration.

This range is ensured, as described above, by the hollow fiber membrane for blood processing according to the present embodiment comprising a hydrophilic polymer, a polysulfone-based resin, and a fat-soluble vitamin, wherein the content A of the hydrophilic polymer to the total mass of the hydrophilic polymer and the polysulfone-based resin in the whole of the hollow fiber membrane is 3% by mass or larger and 10% by mass or smaller, the abundance ratio B of the hydrophilic polymer to the total mass of the hydrophilic polymer and the polysulfone-based resin in the inner surface of the hollow fiber membrane is 35% by mass or larger and 50% by mass or smaller, and the abundance of the fat-soluble vitamin in the entire surface of the hollow fiber membrane is 0.5 mg or larger and 25 mg or smaller based on 1 g of the hollow fiber membrane.

In the present embodiment, the content of nitric acid ions can be measured by a method described later in Examples.

[Methods for producing hollow fiber membrane for blood processing and hollow fiber membrane-based blood processing apparatus]

Methods for producing the hollow fiber membrane for blood processing and the hollow fiber membrane-based blood processing apparatus according to the present embodiment will be described.

(Process of producing hollow fiber membrane for blood processing)

As shown in the flow chart of Figure 9, the hollow fiber membrane for blood processing can be produced through a dope preparation step, an internal coagulant preparation step, a spinning step, a coagulation step, a rinsing step, a drying step, a rolling-up step, and a bundling and cutting step.

Each of these steps will be described in detail with reference to Figure 10.

(Dope preparation step)

The dope preparation step involves dissolving a polysulfone-based resin and a hydrophilic polymer in a solvent to prepare a dope 231. A hydrophilic polymer having a molecular weight of 300,000 or larger is preferably used. A hydrophilic polymer having a molecular weight of 850,000 or larger is more preferably used. Among these hydrophilic polymers, polyvinylpyrrolidone is particularly preferably used. Examples of the solvent may include dimethylacetamide (DMAC), N-methyl-2-pyrrolidone (NMP), and dimethyl sulfoxide (DMSO).

In the case of adding the hydrophilic polymer to the dope, the amount of the hydrophilic polymer having a molecular weight of 300,000 or larger is preferably 10% by mass to 30% by mass, more preferably 14% by mass to 27% by mass, further preferably 15% by mass to 26% by mass, particularly preferably 16% by mass to 25% by mass, in terms of the blending ratio of the hydrophilic polymer to the polysulfone-based resin.

(Internal coagulant preparation step)

The internal coagulant preparation step involves preparing a solvent/nonsolvent mixture of a polysulfone-based resin to prepare an internal coagulant 232. Examples of the solvent may include DMAC, NMP, and DMSO. Examples of the nonsolvent may include water and alcohols. The composition of the dope, coagulation conditions, etc. may be appropriately adjusted, thereby obtaining the hollow fiber membrane according to the present embodiment wherein the abundance ratio B of the hydrophilic polymer is controlled in a particular range.

(Spinning step)

The spinning step involves, as shown in Figure 10, performing spinning by discharging the dope from the outside tube of an annular slit nozzle 233 having a double tube structure while discharging the internal coagulant from the inside tube. The extruded dope is allowed to travel a distance of, for example, 5 cm to 1 m in air and then dipped in a coagulation bath 234.

Preferably, the extruded dope is sufficiently coagulated before being dipped in the coagulation bath 234. In this context, the amounts of the dope 231 and the internal coagulant 232 discharged may be appropriately adjusted in order

to obtain a hollow fiber membrane having the desired membrane thickness and inner diameter.

(Coagulation step)

The dope extruded from the annular slit nozzle 233 is dipped in the coagulation bath 234.
From the viewpoint of more sufficiently coagulating the extruded dope and sufficiently eluting the internal coagulant, water of 40°C to 70°C is preferably used in the coagulation bath. The dipping rate of the extruded dope is preferably 10 cm/min to 100 cm/min.

(Rinsing, drying, and rolling-up steps)

The hollow fiber membrane 230 thus dipped in the coagulation bath is washed, if necessary, in a rinsing bath 235, then dried in a dryer 236 set to an internal temperature of, for example, 100°C to 180°C, and rolled up using a take-up roller 237. The hollow structure of the hollow fiber membrane is formed in these coagulation and rinsing steps, which elute only the internal coagulant by way of water or the like. The hollow fiber membrane 230 thus obtained may be further washed with hot water or the like for removal of unwashed solvent residues and may be dried after attachment thereto of a pore size keeping agent such as glycerin, if necessary.

(Bundling and cutting step)

After the rolling-up step, the obtained hollow fiber membrane bundle is cut. In this way, a hollow fiber membrane bundle 238 comprising a plurality of hollow fiber membranes can be produced.
The hollow fiber membrane for blood processing according to the present embodiment can be produced, as described above, by use of a dry/wet technique of membrane formation known in the art.
In the dope preparation step, the amounts of the polysulfone-based polymer and the hydrophilic polymer added are adjusted in order to adjust the content A of the hydrophilic polymer to 3% by mass or larger and 10% by mass or smaller.
Examples of a common solvent in the dope may include those described above as well as solvents such as sulfolane and dioxane and mixed solvents of two or more of these solvents. The dope may be supplemented with an additive such as water in order to control the pore size of the hollow fiber membrane of interest.
In the process of forming the hollow fiber membrane, a tube-in-orifice-type spinneret may be used as the annular slit nozzle 233 having a double tube structure. The dope and a bore liquid for coagulating the dope are simultaneously discharged into air from the orifice of the spinneret and from a tube, respectively. Water or a coagulant composed mainly of water can be used as the bore liquid. Its composition or the like may be determined according to the permeability of the hollow fiber membrane of interest. In general, a mixed solution of water with the solvent used in the dope is preferably used. For example, an aqueous solution containing 0 to 65% by mass of DMAC is used. Hydrophilic polymers may be further added to the bore liquid to adjust the abundance of the hydrophilic polymer in the membrane surface.
The dope discharged from the spinneret together with the bore liquid is allowed to travel an air gap portion and introduced and dipped in the coagulation bath 234 (composed mainly of water) placed below the spinneret to complete coagulation. As described above, the hollow fiber membrane bundle is obtained through the rinsing, drying, and rolling-up steps. Alternatively, the hollow fiber membrane may be rolled up after the washing step and then dried to obtain a hollow fiber membrane bundle.

(Process of producing hollow fiber membrane-based blood processing apparatus)

The hollow fiber membrane-based blood processing apparatus according to the present embodiment can be produced as follows: the bundle of the hollow fiber membranes for blood processing is inserted to a tubular container having an inlet and an outlet for a predetermined fluid as a liquid to be processed; a potting agent such as polyurethane is injected into both ends of the bundle to form potting layers; after sealing of both ends and subsequent curing, the redundant potting agent is removed by cutting so that the hollow portion is open at both ends; and headers having an inlet and an outlet for the fluid are attached thereto.
Then, the hollow fiber membrane-based processing apparatus according to the present embodiment is subjected to a step of injecting a liquid as described later in order to control the content A of the hydrophilic polymer, the abundance ratio B of the hydrophilic polymer, and the abundance of the fat-soluble vitamin in predetermined ranges, and then further subjected to a step of removing the solvent by drying and a step of radiation-sterilizing the apparatus.

<Step of injecting liquid>

A mixed solution of the fat-soluble vitamin and the hydrophilic polymer (coating solution) is injected to a blood

distribution surface of the hollow fiber membrane to control the abundance of the fat-soluble vitamin in the entire surface of the hollow fiber membrane to 0.5 mg or larger and 25 mg or smaller based on 1 g of the hollow fiber membrane, the abundance ratio B of the hydrophilic polymer in the inner surface of the hollow fiber membrane to 35% by mass or larger and 50% by mass or smaller, and the content A of the hydrophilic polymer in the whole of the hollow fiber membrane to 3% by mass or larger and 10% by mass or smaller.

The rate of attachment of the coating solution can be controlled by appropriately selected or combined method(s), for example, a method which involves adjusting the concentration of the mixed solution, a method which involves changing the duration of the contact between the injected coating solution and the membrane or the injection pressure or temperature of the coating solution, thereby adjusting the amount of the coating solution permeating the membrane, and/or a method which involves adjusting the rate of solution drainage by air blow or the like after attachment of the mixed solution.

The abundance of the fat-soluble vitamin can be controlled without particular problems by these methods. By contrast, more careful conditions are required for the immobilization of the hydrophilic polymer. Specifically, the mere coating of a hydrophilic polymer solution cannot achieve the abundance ratio B of the hydrophilic polymer in the inner surface of the hollow fiber membrane, which is necessary for exerting the effects of the present invention, presumably because the originally residing hydrophilic polymers and the additional hydrophilic polymers are insufficiently intertwined.

The present inventors have conducted diligent studies and consequently found that the following methods are useful therefor.

The first method involves injecting approximately 100 to 40% aqueous alcohol solution or the like before injection of the mixed solution of the fat-soluble vitamin and the hydrophilic polymer.

The second method employs 65% or higher aqueous alcohol solution as the mixed solution of the fat-soluble vitamin and the hydrophilic polymer.

The third method involves setting the temperature of the mixed solution of the fat-soluble vitamin and the hydrophilic polymer to be contacted with the membrane to a relatively high temperature, for example, the range of 40 to 70°C.

Any of these methods are effective for swelling the originally residing hydrophilic polymers and easily intertwining the additional hydrophilic polymers therewith.

These methods may be adopted alone or in combination.

<Step of removing solvent by drying>

The step of removing, by drying, the solvent in the solution used in the step of injecting a liquid may involves aerating the apparatus with gas such as air or nitrogen or drying the apparatus in vacuum. Any method can be used for this purpose.

The temperature in this step is not particularly limited.

<Step of radiation-sterilizing apparatus>

The hollow fiber membrane-based blood processing apparatus is sterilized by exposure to radiation.

The radiation sterilization method can employ electron beam, gamma rays, x-rays, and the like, any of which may be used.

In the present embodiment, the exposure dose of radiation is 15 to 50 kGy for $\gamma$-rays or electron beam. The apparatus is more preferably exposed to radiation in a dose range of 20 to 40 kGy. Upon radiation sterilization under such conditions, the hydrophilic polymer constituting the hollow fiber membrane can be partially cross-linked, thereby preventing the hydrophilic polymer being from eluted with favorable blood compatibility maintained.

## Examples

Hereinafter, the present invention will be described with reference to specific Examples and Comparative Examples. However, the present invention is not limited by these Examples shown below.

First, various assay methods used in Examples will be described.

[Abundance of fat-soluble vitamin in entire surface of hollow fiber membrane]

A specific method for measuring the abundance of the fat-soluble vitamin present in the entire surface of the hollow fiber membrane will be shown below.

The hollow fiber membrane-based blood processing apparatus was disassembled to collect the hollow fiber membranes, which were in turn rinsed and then vacuum-dried at 40°C.

4 g of the hollow fiber membranes thus dried was weighed into a vial. 80 mL of an aqueous solution containing

1% by mass of polyethylene glycol-t-octyl ether was added thereto. The fat-soluble vitamin was extracted at room temperature for 60 minutes under ultrasonic vibration.

The fat-soluble vitamin was quantified by liquid chromatography. The amount of the fat-soluble vitamin in the extract was determined using a calibration curve obtained from the peak area of a standard fat-soluble vitamin solution.

Specifically, a column (Shodex Asahipak ODP-506E packed column for HPLC) was attached to a high-performance liquid chromatography apparatus (pump: PU-1580 manufactured by JASCO Corp., detector: RID-6A manufactured by Shimadzu Corp., autoinjector: SIL-6B manufactured by Shimadzu Corp., data processing: GPC-8020 manufactured by Tosoh Corp., column oven: 556 manufactured by GL Sciences Inc.). A mobile phase methanol for high-performance liquid chromatography was applied to the column (temperature: 40°C) at a flow rate of 1 mL/min. The fat-soluble vitamin concentration was determined from the area of an absorption peak in the ultraviolet region.

From this concentration, the mass (mg/g) of the fat-soluble vitamins present in the entire surface of the hollow fiber membrane was determined with extraction efficiency as 100%.

In this context, the abundance of the fat-soluble vitamin in the entire surface of the hollow fiber membrane was determined, also including the amount of partially oxidized fat-soluble vitamins. In order to determine the amount of the partially oxidized fat-soluble vitamins, fat-soluble vitamins used for calibration curve preparation were exposed in advance to 50 kGy radiation in air, and a group of peaks for use in area calculation was determined beforehand.

[Mass ratio of hydrophilic polymer to total mass of hydrophilic polymer and polysulfone-based resin in whole of hollow fiber membrane for blood processing (content A)]

The mass ratio of the hydrophilic polymer to the total mass of the hydrophilic polymer and the polysulfone-based resin in the whole of the hollow fiber membrane for blood processing, i.e., the content A (represented by content A of hydrophilic polymer in whole of hollow fiber membrane in Tables 1 and 2 below), was determined as described below.

The hollow fiber membrane was dissolved at a sample concentration of 3% by mass in deuterated chloroform (manufactured by In-Situ Oxidative Technologies, Inc. (ISOTEC)). The solution was filtered through glass wool. This sample was assayed at room temperature using a nuclear magnetic resonance apparatus Avance 600 (manufactured by Bruker BioSpin K.K.).

Observation frequency was set to 600 MHz, and chemical shifts were based on TMS (= 0.00 ppm).

On the basis of the obtained NMR chart, the molar ratio between the polysulfone-based resin and the hydrophilic polymer was determined from the integrated strength of peaks derived from protons of a group specific for the polysulfone-based resin and the integrated strength of peaks derived from the protons of a group specific for the hydrophilic polymer. The obtained molar ratio was converted to a mass ratio to calculate the mass ratio of the hydrophilic polymer to the total mass of the hydrophilic polymer and the polysulfone-based resin in the whole of the hollow fiber membrane.

[Mass ratio of hydrophilic polymer to total mass of hydrophilic polymer and polysulfone-based resin in inner surface of hollow fiber membrane for blood processing (abundance ratio B)]

The mass ratio of the hydrophilic polymer to the total mass of the hydrophilic polymer and the polysulfone-based resin in the inner surface of the hollow fiber membrane for blood processing, i.e., the abundance ratio B (represented by abundance ratio (B) of hydrophilic polymer in inner surface of hollow fiber membrane in Tables 1 and 2 below), was determined as described below.

The hollow fiber membrane-based blood processing apparatus was disassembled to recover the hollow fiber membranes.

The hollow fiber membranes were strapped to prepare a bundle having approximately 50 filaments x 20 cm, which was in turn dipped overnight in a vat filled with distilled water. This vat was overflowed with distilled water by replenishing fresh distilled water at all times.

The hollow fiber membrane bundle was recovered, then cut into 5 cm, and frozen in a freezer of -40°C. The hollow fiber membranes were freeze-dried overnight at a vacuum of approximately 0.3 to 0.4 torr. The dried hollow fiber membranes were cut open in the longitudinal direction to expose the inner surface. Several filaments were arranged on a double-faced tape to prepare a sample. The sample was assayed using an X-ray photoelectron spectroscopy apparatus (manufactured by Thermo Fisher Scientific K.K., ESCALAB 250) under the following conditions:

Assay conditions

Excitation source: mono. AlK$\alpha$ 15 kV x 10 mA Scanning range
Survey scan: 0 to 1,100 eV
Narrow scan: C1s, O1s, N1s, and S2p
Pass Energy: 100 eV

From the area strength of the obtained Narrow Scan spectrum, the element concentrations were determined using relative sensitivity factors in the library of the apparatus, followed by quantitative calculation. The relative sensitivity factors used were C1s: 0.296, O1s: 0.711, S2p: 0.666, and N1s: 0.477.

Hereinafter, a hollow fiber membrane containing polysulfone represented by the formula (1) as the polysulfone-based resin and polyvinylpyrrolidone as the hydrophilic polymer will be described as an example.

S2p obtained by assay is derived from polysulfone, and N1s is derived from polyvinylpyrrolidone. The formula weight of the repeat unit of polysulfone is 442, and the formula weight of the repeat unit of polyvinylpyrrolidone is 111. The abundance ratio B of polyvinylpyrrolidone in the inner surface of the hollow fiber membrane can be determined according to the following expression wherein S represents the element concentration (atomic %) of S2p, and N represents the element concentration (atomic %) of N1s:

[Expression 2]

$$\frac{111 \times N}{(111 \times N) + (442 \times S)} \times 100 \quad \text{(\% by mass)}$$

Hereinafter, the total abundance ratio of hydrophilic polymers in the inner surface of a hollow fiber membrane containing polysulfone represented by the formula (1) as the polysulfone-based resin and polyvinylpyrrolidone and polyethylene glycol as the hydrophilic polymers, i.e., the total abundance ratio of polyvinylpyrrolidone and polyethylene glycol in the inner surface of the hollow fiber membrane, will be described.

As in the hollow fiber membrane containing only polyvinylpyrrolidone as the hydrophilic polymer, the element concentrations were determined using the X-ray photoelectron spectroscopy apparatus, followed by quantitative calculation.

S2p obtained by assay is derived from polysulfone, and N1s is derived from polyvinylpyrrolidone. O1s is derived from four oxygen atoms in the polysulfone repeat unit, one oxygen atom in the polyvinylpyrrolidone repeat unit, and one oxygen atom in the polyethylene glycol repeat unit. The formula weight of the repeat unit of polysulfone is 442; the formula weight of the repeat unit of polyvinylpyrrolidone is 111; and the formula weight of the repeat unit of polyethylene glycol is 44. The abundance ratio B of the hydrophilic polymers (polyvinylpyrrolidone + polyethylene glycol) in the inner surface of the hollow fiber membrane can be determined according to the following expression wherein S represents the element concentration (atomic %) of S2p; N represents the element concentration (atomic %) of N1s; and O represents the element concentration (atomic %) of O1s:

[Expression 3]

$$\frac{(111 \times N) + (O - 4 \times S - N) \times 44}{(111 \times N) + (442 \times S) + (O - 4 \times S - N) \times 44} \times 100 \quad \text{(\% by mass)}$$

[Antioxidative capacity]

The antioxidative capacity of the hollow fiber membrane-based blood processing apparatus was determined by the following method:

First, ferric chloride hexahydrate was dissolved in pure water to prepare 0.3 w/v% (amount (g) of solute in 100 mL of solution) aqueous solution.

The hollow fiber membrane-based blood processing apparatus was disassembled to collect the hollow fiber membranes, which were in turn rinsed and then vacuum dried at 40°C.

1 g of the hollow fiber membranes thus dried and 20 mL of the aqueous ferric chloride solution were weighed into a vial, degassed at 60 mmHg for 10 minutes, and then incubated at 30°C for 4 hours under shaking (the fat-soluble vitamin present in the surface of the hollow fiber membrane reduced iron (III) ions into iron (II)).

2.6 mL of the incubated aqueous solution was mixed with 0.7 mL of ethanol and 0.7 mL of 0.5 w/v% aqueous

ethanol solution of 2,2'-bipyridyl separately prepared, and the mixture was incubated at 30°C for 30 minutes under shaking (iron (II) and bipyridyl formed a complex to develop color).

The absorbance of the colored solution was measured at 520 nm using a spectrometer.

The same incubation, color reaction, and absorbance measurement as above were performed using an ethanol solution of a fat-soluble vitamin with a known concentration instead of the hollow fiber membrane to prepare a calibration curve. The antioxidative capacity exhibited by 1 g of the hollow fiber membrane was determined as a value based on the mass of the fat-soluble vitamin.

The hollow fiber membrane-based blood processing apparatus was evaluated such that the antioxidative capacity was determined to be favorable (indicated by a circle) when the value based on the mass of the fat-soluble vitamin present in the surface of the hollow fiber membrane was 0.4 mg or higher based on 1 g of the hollow fiber membrane, and determined to be unfavorable (indicated by a cross mark) when this value was lower than 0.4 mg.

[Determination of lactate dehydrogenase (LDH) activity]

The blood compatibility of the hollow fiber membrane was evaluated on the basis of platelet attachment to the surface of the hollow fiber membrane and quantified with the activity of lactate dehydrogenase (LDH) contained in the platelet attached to the hollow fiber membrane as an index.

The hollow fiber membrane-based blood processing apparatus was disassembled to collect the hollow fiber membranes for blood purification, both ends of which were in turn bonded using an epoxy adhesive such that an effective length was 15 cm and the inner surface area of the membrane was 50 $mm^2$ (56 filaments for the hollow fiber membrane having an inner diameter of 185 $\mu$m) to prepare a mini module.

This mini module was washed with 3 mL of saline (manufactured by Otsuka Pharmaceutical Co., Ltd., Otsuka Seishoku Chu) flowed at a flow rate of 0.6 mL/min in the inside of the hollow fiber membrane (hereinafter, this procedure is referred to as "priming").

Then, 15 mL of heparin-supplemented human blood was adjusted to a temperature of 37°C and circulated at a flow rate of 1.2 mL/min for 4 hours in the mini module. After the circulation, the inside and outside of the mini module were each washed with 10 mL of saline.

From the mini module thus washed, 56 filaments of the hollow fiber membranes having a length of 7 cm were collected, then chopped, and placed in a Spitz tube for LDH assay to prepare an assay sample.

From the washed mini module, half of all the hollow fiber membranes having a length of 14 cm was collected, then chopped, and placed in a Spitz tube for LDH assay to prepare an assay sample.

Next, Triton X-100 (manufactured by Nacalai Tesque, Inc.) was dissolved in phosphate-buffered saline (PBS) (manufactured by Wako Pure Chemical Industries, Ltd.). 0.5 mL of the obtained solution containing 0.5% by volume of Triton X-100 in PBS was added to the Spitz tube for LDH assay and then centrifuged (2700 rpm x 5 min) to precipitate the hollow fiber membranes in the solution. The cells (mainly, platelet) attached to the hollow fiber membranes were disrupted by extraction for 60 minutes under shaking to extract intracellular LDH. A 0.05 mL aliquot of this extract was collected and reacted by the further addition of 2.7 mL of 0.6 mM sodium pyruvate solution and 0.3 mL of 1.277 mg/mL nicotinamide adenine dinucleotide (NADH) solution. After reaction for at 37°C for additional one hour, the absorbance was measured at 340 nm. Likewise, the absorbance of a blood-unreacted membrane (blank) was measured, and the difference in absorbance therebetween was calculated according to the expression (IV) shown below.

The value obtained according to the expression (IV) below was divided by an effective membrane area as shown below in the expression (V). The LDH activity was evaluated on the basis of the obtained value.

In this evaluation method, the larger rate of this decrease means higher LDH activity, i.e., the larger amount of the platelet attached to the surface of the hollow fiber membrane. The hollow fiber membrane was determined to have favorable blood compatibility (indicated by a circle) when the LDH activity was 50 or less, and determined to have poor blood compatibility (indicated by a cross mark) when the LDH activity exceeded 50.

$$\Delta 340\text{ nm} = \text{Absorbance of the sample after 60 minutes} - \text{Absorbance of the blank after 60 minutes} \quad \ldots \text{(IV)}$$

$$\text{LDH activity} = \Delta 340\text{ nm} / \text{Effective membrane area} \quad \ldots \text{(V)}$$

Figure 11 shows the relationship between the amount of the fat-soluble vitamin in the entire surface of the

hollow fiber membrane and the LDH activity when the abundance ratio B of the hydrophilic polymer in the inner surface of the hollow fiber membrane (mass ratio of the hydrophilic polymer to the total mass of the hydrophilic polymer and the polysulfone-based resin in the inner surface of the hollow fiber membrane for blood processing) is 35 to 50% by mass.

In Figure 11, the unit (mg/gHF) indicated in the abscissa means the abundance (mg) of the fat-soluble vitamin in the entire surface of the hollow fiber membrane based on 1 g of the hollow fiber membrane.

These results demonstrated that the amount of the fat-soluble vitamin of 0.5 mg or larger and 25 mg or smaller based on 1 g of the hollow fiber membrane resulted in favorable LDH activity, whereas the amount of 0.4 mg (less than 0.5 mg) or 27 mg (exceeding 25 mg) that fell outside this range resulted in unfavorable LDH activity.

Figure 12 shows the relationship between the abundance ratio B of the hydrophilic polymer in the inner surface of the hollow fiber membrane and the LDH activity when the abundance of the fat-soluble vitamin in the entire surface of the hollow fiber membrane is 0.5 to 25 mg based on 1 g of the membrane. These results demonstrated that the LDH activity satisfied the goal of the present invention when the abundance ratio B of the hydrophilic polymer in the inner surface of the hollow fiber membrane was 35% by mass or larger, but did not satisfy the goal when the abundance ratio B was less than 35% by mass.

[Evaluation of priming performance (quantification of residual quantity of air)]

An experimental apparatus configured as shown in Figure 13 was used to measure and evaluate the residual quantity of air after priming.

The experimental apparatus of Figure 13 is equipped with a container 122 containing saline, a hollow fiber membrane-based blood processing apparatus 121, and a container 124.

The hollow fiber membrane-based blood processing apparatus 121 is fixed so that its center is aligned with the intermediate position between the water surfaces of the container 122 and the container 124.

A commercially available blood circuit for dialysis was cut into an appropriate length and used as a tube 125. Saline is sent into the hollow fiber membrane-based blood processing apparatus 121 via the tube 125 from the container 122. In addition, a processing liquid is sent into the container 124 via a predetermined tube. An elevation difference (head difference) 126 from the container 122 to the container 124 was set to 100 cm.

First, the tube 125 was packed with saline, which was then injected from the container 122 through the hollow fiber membrane-based blood processing apparatus 121 into the container 124 by means of the head difference 126. The first processing liquid flowed out of the container 124, and then, 500 mL of saline was injected into the apparatus only by the drive force based on the head difference.

The hollow fiber membrane-based blood processing apparatus was left standing without application of impact or vibration from the start of injection until 450 mL of the saline was consumed. While the remaining 50 mL of the saline was injected into the hollow fiber membrane-based blood processing apparatus, the apparatus was tapped to allow only air residing in the upper header to flow into the tube.

Next, ABP-01 pump manufactured by Asahi Kasei Medical Co., Ltd. was placed at a point 127 for pump placement. In order to eject air, the liquid was injected into the hollow fiber membrane-based blood processing apparatus at a flow rate of 600 mL/min under tapping and vibration until air was no longer ejected.

The ejected air was collected into a graduated cylinder. The scale was read and recorded as the residual quantity (mL) of air.

The hollow fiber membrane-based blood processing apparatus was evaluated such that a residual quantity of air less than 5 mL was determined to be not problematic for practical use (indicated by a circle) and a residual quantity of air of 5 mL or larger was determined to have a risk of sending air into patient's body beyond a blood chamber (indicated by a cross mark).

[Effluent from hollow fiber membrane]

The passages inside and outside the hollow fiber membranes of the hollow fiber membrane-based blood processing apparatus were each washed with 1000 mL of distilled water (flow rate: 100 mL/min) and drained by air blow.

Subsequently, the hollow fiber membrane-based blood processing apparatus was disassembled to recover 1.5 g of the hollow fiber membranes, which was in turn subjected to 1-hour extraction in 150 mL of pure water of 70°C.

The UV spectrum of the extract was measured at 350 nm to 220 nm. The absorbance that exhibited the maximum absorption was used as an index substituted for the amount of effluents from the hollow fiber membrane.

The hollow fiber membrane-based blood processing apparatus was evaluated such that effluents were determined to be not problematic for practical use (indicated by a circle) when the absorbance was lower than 0.10, and determined to be problematic for practical use (indicated by a cross mark) when the absorbance was 0.10 or higher.

[Toughness of hollow fiber membrane]

In a room having room temperature (20 to 25°C) and a humidity of 55 to 60 RH%, one dried 20-cm hollow fiber membrane was fixed using a chuck in a tensile tester manufactured by Shimadzu Corp. (EZ Test series) and stretched at a rate of 30 cm/min. Its stress at break (gf) was measured.

The elongation at break of the hollow fiber membrane was divided by the length 20 cm before measurement of the hollow fiber membrane and then multiplied by 100. The obtained value was defined as the degree of elongation (%). The toughness was calculated according to the following expression:

$$\text{Toughness (gf}\cdot\text{\%)} = \text{Stress at break (gf)} \times \text{Degree of elongation (\%)}$$

The hollow fiber membrane was evaluated such that toughness of 1000 gf.% or higher was determined to be not problematic for practical use (indicated by a circle) and toughness lower than 1000 gf.% was determined to be problematic for practical use (indicated by a cross mark).

[Stability test on permeability in harsh environment (assay of permeability before and after storage at high temperature)]

The hollow fiber membrane-based blood processing apparatuses (effective membrane area: 1.5 $m^2$) of Examples and Comparative Examples described later were each heat-treated for 6 weeks in a thermostat bath of 60°C.

The permeability (ultrafiltration rate) of each hollow fiber membrane-based blood processing apparatus was assayed before and after the heat treatment according to a method prescribed by ISO8637:2004 5.6.3 (or JIS T3250:2005 5.6.3) except that the test solution was changed from bovine blood to distilled water. Specifically, the assay was conducted by the following method:

(1) A test circuit was assembled as shown in Figure 14.
Drip chambers 140 to 142, a pressure regulator 131, a hollow fiber membrane-based blood processing apparatus 132, a filtrate recovery container 133, a test solution reservoir 134, a blood pump 135, and a waste liquid recovery container 136 were connected using predetermined tubes as shown in Figure 14.
A blood nozzle inlet pressure gauge 138, a blood nozzle outlet pressure gauge 137, and a filtrate-side pressure gauge 139 were disposed in the drip chambers 140, 141, and 142, respectively.
A commercially available blood circuit for dialysis was cut into an appropriate length and applied to the tubes for connection.
The hollow fiber membrane-based blood processing apparatus 132 was placed in the longitudinal direction (direction in which nozzles for the blood-side passage are vertically arranged). A circuit-unconnected nozzle for the dialysis solution-side passage was stoppered.
As indicated by a broken line 143 in Figure 14, the liquid level of the drip chamber 140 and the liquid feed ports of the drip chambers 141 and 142 are adjusted to be the same height as the center of the hollow fiber membrane-based blood processing apparatus 132.
(2) Distilled water incubated at 37°C was used as a test solution.
(3) The blood pump 135 and the pressure regulator 131 were adjusted such that the flow rate into the blood nozzle was 200 mL/min and the transmembrane pressure (TMP) was 50 mmHg to stabilize the blood circuit and the flow of the filtrate.
In this context, TMP was calculated according to the following expression:

$$\text{TMP} = \{(\text{Pressure value of the blood nozzle outlet pressure gauge 137}) + (\text{Pressure value of the blood nozzle inlet pressure gauge 138})\} / 2 - (\text{Pressure value of the filtrate-side pressure gauge 139})$$

(4) The amount of the filtrate per hour was measured, and the ultrafiltration rates before and after the heat treatment were each calculated according to the following expression:

$$(\text{Ultrafiltration rate: UFR}) = (\text{Amount of the filtrate}) / \text{TMP (unit: mL/(hr·mmHg))}$$

The hollow fiber membrane-based blood processing apparatus was evaluated such that the stability was determined to be not problematic for practical use (indicated by a circle) when the rate of change in UFR between before and after the heat treatment was within ±10%, and determined to be problematic for practical use (indicated by a cross mark) when the rate of change in UFR exceeded ±10%. Also, the hollow fiber membrane-based blood processing apparatus was evaluated such that the apparatus was determined to be able to maintain high membrane performance (indicated by a circle) when UFR after the heat treatment exceeded 40 mL/(hr·mmHg), and determined to be unable to do so (indicated by a cross mark) when UFR after the heat treatment was lower than the value.

[Measurement of concentration of nitric acid ion contained in hollow fiber membrane]

Approximately 1 g of a sample was precisely weighed (mass of the hollow fiber membrane before extraction) for sampling and placed in a centrifuge tube (IWAKI cat. No. 2345-050; Asahi Glass Co., Ltd.) made of polypropylene.
50 mL of distilled water (046-16971 for high-performance liquid chromatography; Wako Pure Chemical Industries, Ltd.) was added thereto, and the tube was sonicated (Elmasonic S30H; Pathtech Pty Ltd.) for 20 minutes. Nitric acid ions were quantified by ion chromatography under conditions shown below. The same procedures as above were performed without the sample to prepare a blank sample.
The sample after the extraction operation was drained at room temperature and then vacuum-dried at 60°C for 12 hours, and its weight was measured (mass of the hollow fiber membrane after extraction). The nitric acid ion concentration was converted to that with respect to the mass of the hollow fiber membrane after extraction from the value quantified by ion chromatography.

Ion chromatography conditions

Apparatus: IC-2001 manufactured by Tosoh Corp.
Column: TSKgel Super IC-AZ
Eluent: 6.3 mM $NaHCO_3$ + 1.7 mM $NaCO_3$
Flow rate: 0.8 mL/min
Pressure: 9.0 mPa

[Example 1]

17 parts by mass of PSf (manufactured by Solvay Advanced Polymers, LLC., P-1700)
4 parts by mass of PVP (manufactured by ISP Ltd., K-90)
79 parts by mass of dimethylacetamide (hereinafter, referred to as DMAC)

These components were used to prepare a uniform dope.
In this context, PSf represents a polysulfone resin, and PVP represents polyvinylpyrrolidone.
An aqueous solution containing 42% by mass of DMAC was used as a bore liquid and discharged from a spinneret together with the dope.
The amounts of the dope and the bore liquid discharged were adjusted such that the membrane thickness and the inner diameter were 45 μm and 185 μm, respectively, after drying.
The discharged dope was dipped in a coagulation bath (consisting of water) of 60°C positioned 50 cm below the spinneret. After a coagulation step at a rate of 30 m/min and a rinsing step (rinsing treatment), the hollow fiber membranes were introduced to a hot-air dryer, dried at 120°C for 2 minutes, and further heat-treated at 160°C for 0.5 minutes. Then, the crimped polysulfone-based hollow fiber membranes were rolled up.
Next, the rolled-up hollow fiber membrane bundle of 10000 filaments was loaded in a plastic tubular container designed such that the hollow fiber membrane had an effective membrane area of 1.5 $m^2$. Both ends thereof were fixed by bonding with urethane resins. The surfaces at both ends were cut so that the hollow fiber membranes were open at both ends.

PVP and α-tocopherol (manufactured by Wako Pure Chemical Industries, Ltd., special grade) were dissolved at concentrations of 0.05% by mass and 0.5% by mass, respectively, in an aqueous solution consisting of 65 parts by mass of 2-propanol (manufactured by Wako Pure Chemical Industries, Ltd., special grade) and 35 parts by mass of distilled water (Otsuka Pharmaceutical Co., Ltd.), and the resulting coating solution was heated to 40°C and then injected (120 mL) into the hollow fiber membranes from the open end, followed by flash with 0.3 MPa air for 10 seconds. Subsequently, the follow fiber membranes were dried for 1 hour using dry air of 40°C. After the drying, header caps were attached to both ends. Blood feed- and exit-side nozzles were stoppered. The resulting module was then exposed to 25 kGy electron beam to obtain a hollow fiber membrane-type blood processing apparatus having an effective membrane area of 1.5 $m^2$.

In this hollow fiber membrane-type blood processing apparatus, the abundance of the fat-soluble vitamin (α-tocopherol) in the entire surface of the hollow fiber membrane was 7 mg per g of the hollow fiber membrane, the abundance ratio B of the hydrophilic polymer (PVP) in the inner surface of the hollow fiber membrane was 44% by mass, and the content A of the hydrophilic polymer (PVP) in the whole of the hollow fiber membrane was 3% by mass.

Results of assaying its properties are shown in Table 1 below.

[Example 2]

Before injection of the coating solution into the hollow fiber membrane, 120 mL of an aqueous solution consisting of 80 parts by mass of 2-propanol and 20 parts by mass of distilled water was injected into the membranes, followed by flash with 0.3 MPa air for 10 seconds.

The α-tocopherol concentration of the coating solution was set to 0.05% by mass.

The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 0.5 mg per g of the membrane, the abundance ratio B of the hydrophilic polymer (PVP) in the inner surface of the hollow fiber membrane was 46% by mass, and the content A of the hydrophilic polymer (PVP) in the whole of the hollow fiber membrane was 3% by mass.

Results of assaying its properties are shown in Table 1 below.

[Example 3]

The α-tocopherol concentration of the coating solution was set to 0.1% by mass.

The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 1.5 mg per g of the membrane, the abundance ratio B of the hydrophilic polymer (PVP) in the inner surface of the hollow fiber membrane was 46% by mass, and the content A of the hydrophilic polymer (PVP) in the whole of the hollow fiber membrane was 3% by mass.

Results of assaying its properties are shown in Table 1 below.

[Example 4]

The α-tocopherol concentration of the coating solution was set to 1.2% by mass.

25 KGy gamma rays were used in the exposure instead of 25 KGy electron beam.

The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 1.5 mg per g of the membrane, the abundance ratio B of the hydrophilic polymer (PVP) in the inner surface of the hollow fiber membrane was 46% by mass, and the content A of the hydrophilic polymer (PVP) in the whole of the hollow fiber membrane was 3% by mass.

Results of assaying its properties are shown in Table 1 below.

[Example 5]

The α-tocopherol concentration of the coating solution was set to 1.6% by mass.

Before exposure to electron beam, nitrogen gas was circulated for 10 minutes from the blood feed- and exit-side nozzles of the blood processing apparatus. The other conditions were the same as those of Example 4 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 25 mg per g of the membrane, the abundance ratio B of the hydrophilic polymer (PVP) in the inner surface of the hollow fiber membrane was 43% by mass, and the content A of the hydrophilic polymer (PVP) in the whole of the hollow fiber membrane was 3% by mass.
Results of assaying its properties are shown in Table 1 below.

[Example 6]

The PVP concentration of the coating solution was set to 0.01% by mass.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 1.5 mg per g of the membrane, the abundance ratio B of the hydrophilic polymer (PVP) in the inner surface of the hollow fiber membrane was 46% by mass, and the content A of the hydrophilic polymer (PVP) in the whole of the hollow fiber membrane was 3% by mass.
Results of assaying its properties are shown in Table 1 below.

[Example 7]

The PVP concentration of the coating solution was set to 0.02% by mass.
The exposure dose of electron beam was set to 15 kGy.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 39% by mass, and the content A of the hydrophilic polymer (PVP) in the whole of the hollow fiber membrane was 3% by mass.
Results of assaying its properties are shown in Table 1 below.

[Example 8]

The PVP concentration of the coating solution was set to 0.1% by mass.
The exposure dose of electron beam was set to 40 kGy.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 40% by mass, and the content A of the hydrophilic polymer (PVP) in the whole of the hollow fiber membrane was 3% by mass.
Results of assaying its properties are shown in Table 1 below.

[Example 9]

The PVP concentration of the coating solution was set to 0.18% by mass.
The exposure dose of electron beam was set to 50 kGy.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 50% by mass, and the content A of the hydrophilic polymer (PVP) in the whole of the hollow fiber membrane was 3% by mass.
Results of assaying its properties are shown in Table 1 below.

[Example 10]

For the dope, the amount of PVP added was set to 9 parts by mass, and the amount of dimethylacetamide added was set to 74 parts by mass.
The other conditions were the same as those of Example 1 to obtain a hollow fiber membrane-type blood

processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 39% by mass, and the content A of the hydrophilic polymer (PVP) in the whole of the hollow fiber membrane was 10% by mass.
Results of assaying its properties are shown in Table 1 below.

[Comparative Example 1]

The α-tocopherol concentration of the coating solution was set to 0.03% by mass.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 0.4 mg per g of the membrane, the abundance ratio B of the hydrophilic polymer (PVP) in the inner surface of the hollow fiber membrane was 44% by mass, and the content A of the hydrophilic polymer (PVP) in the whole of the hollow fiber membrane was 3% by mass.
Results of assaying its properties are shown in Table 2 below.
The obtained hollow fiber membrane-type blood processing apparatus had low antioxidative capacity, unfavorable blood compatibility as is evident from high LDH activity, poor productivity attributed to the high rate of misdetection in the leak test, and insufficiently stable performance under harsh conditions.

[Comparative Example 2]

The α-tocopherol concentration of the coating solution was set to 1.8% by mass.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 27 mg per g of the membrane, the abundance ratio B of the hydrophilic polymer (PVP) in the inner surface of the hollow fiber membrane was 44% by mass, and the content A of the hydrophilic polymer (PVP) in the whole of the hollow fiber membrane was 3% by mass.
Results of assaying its properties are shown in Table 2 below.
The obtained hollow fiber membrane-type blood processing apparatus had unfavorable blood compatibility as is evident from high LDH activity and poor productivity attributed to the high rate of misdetection in the leak test.

[Comparative Example 3]

The PVP concentration of the coating solution was set to 0.008% by mass.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 34% by mass, and the content A of the hydrophilic polymer (PVP) in the whole of the hollow fiber membrane was 3% by mass.
Results of assaying its properties are shown in Table 2 below.
The obtained hollow fiber membrane-type blood processing apparatus had unfavorable blood compatibility as is evident from high LDH activity, poor productivity attributed to the high rate of misdetection in the leak test, and insufficiently stable performance under harsh conditions.

[Comparative Example 4]

The PVP concentration of the coating solution was set to 0.2% by mass.
The other conditions were the same as those of Example 1 to obtain a hollow fiber membrane-type blood processing apparatus.
In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 34% by mass, and the content A of the hydrophilic polymer (PVP) in the whole of the hollow fiber membrane was 3% by mass.
Results of assaying its properties are shown in Table 2 below.

The obtained hollow fiber membrane-type blood processing apparatus had insufficiently stable performance under harsh conditions and was problematic for practical use due to an excessive residual quantity of air after priming and a large amount of effluents.

[Comparative Example 5]

The PVP concentration of the coating solution was set to 0.1% by mass.

The other conditions were the same as those of Example 1 to obtain a hollow fiber membrane-type blood processing apparatus.

In this hollow fiber membrane-type blood processing apparatus, the abundance of the fat-soluble vitamin ($\alpha$-tocopherol) in the entire surface of the hollow fiber membrane was 6 mg per g of the membrane, the abundance ratio B of the hydrophilic polymer (PVP) in the inner surface of the hollow fiber membrane was 32% by mass, and the content A of the hydrophilic polymer (PVP) in the whole of the hollow fiber membrane was 2% by mass.

Results of assaying its properties are shown in Table 2 below.

The obtained hollow fiber membrane-type blood processing apparatus had unfavorable blood compatibility as is evident from high LDH activity, poor productivity attributed to the high rate of misdetection in the leak test, and insufficiently stable performance under harsh conditions, and was problematic for practical use due to a large amount of effluents.

[Comparative Example 6]

For the dope, the amount of PVP added was set to 10 parts by mass, and the amount of dimethylacetamide added was set to 73 parts by mass.

PVP was not added to the coating solution.

The other conditions were the same as those of Example 1 to obtain a hollow fiber membrane-type blood processing apparatus.

In this hollow fiber membrane-type blood processing apparatus, the abundance of the fat-soluble vitamin ($\alpha$-tocopherol) in the entire surface of the hollow fiber membrane was 7 mg per g of the membrane, the abundance ratio B of the hydrophilic polymer (PVP) in the inner surface of the hollow fiber membrane was 33% by mass, and the content A of the hydrophilic polymer (PVP) in the whole of the hollow fiber membrane was 12% by mass.

Results of assaying its properties are shown in Table 2 below.

The obtained hollow fiber membrane-type blood processing apparatus had unfavorable blood compatibility as is evident from high LDH activity, poor productivity attributed to the high rate of misdetection in the leak test, and insufficiently stable performance under harsh conditions, and was problematic for practical use due to low toughness of the hollow fiber membrane, a risk of causing a leak during production or transportation, and a large amount of effluents.

[Comparative Example 7]

The PVP concentration of the coating solution was set to 0.22% by mass, and $\alpha$-tocopherol was not added. The dose of electron beam was set to 50 kGy.

The other conditions were the same as those of Example 1 to obtain a hollow fiber membrane-type blood processing apparatus.

In this hollow fiber membrane-type blood processing apparatus, the abundance of the fat-soluble vitamin ($\alpha$-tocopherol) in the entire surface of the hollow fiber membrane was 0 mg per g of the membrane, the abundance ratio B of the hydrophilic polymer (PVP) in the inner surface of the hollow fiber membrane was 60% by mass, and the content A of the hydrophilic polymer (PVP) in the whole of the hollow fiber membrane was 4% by mass.

Results of assaying its properties are shown in Table 2 below.

The obtained hollow fiber membrane-type blood processing apparatus had low antioxidative capacity, unfavorable blood compatibility as is evident from high LDH activity, poor productivity attributed to the high rate of misdetection in the leak test, and insufficiently stable performance under harsh conditions, and was problematic for practical use due to an excessive residual quantity of air after priming.

[Comparative Example 8]

18.0% by mass of PSf and 4.3% by mass of PVP were dissolved in 77.7% by mass of dimethylacetamide to prepare a dope as a uniform solution. In this context, the blending ratio of PVP to PSf in the dope was 23.9% by mass. This dope was discharged, with its temperature kept at 60°C, from a double annular spinneret together with a bore liquid consisting of a mixed solution containing 30% by mass of dimethylacetamide and 70% by mass of water, then allowed

to travel 0.96 m air gap, dipped in a coagulation bath consisting of water of 75°C, and rolled up at a rate of 80 m/min. For this procedure, the region from the spinneret to the coagulation bath was enclosed in a cylindrical vessel. The internal humidity and temperature of the vessel were controlled at 54.5% and 51°C, respectively, while steam-containing nitrogen gas was circulated in the vessel. The ratio of the air gap to the spinning rate was 0.012 m/(m/min). A rolled-up bundle of 10000 filaments was cut. Then, the resulting bundle was washed over 2 hours by shower with hot water of 80°C from above the cut surface of the bundle to remove solvent residues in the membranes. The resulting membranes were loaded in a tubular container having two nozzles for liquid feed and exit. Both ends thereof were embedded in urethane resins. The cured urethane portions were cut so that the hollow fiber membranes were open at both ends. Both of these open ends were loaded with header caps each having a nozzle for blood feed or exit to assemble a hollow fiber membrane-type blood processing apparatus.

Next, 0.5% by mass of α-tocopherol was dissolved in an aqueous solution containing 57% by mass of IPA, and the resulting coating solution was injected into the bores of the hollow fiber membranes for 52 seconds from the blood feed nozzle of the hollow fiber membrane-type blood processing apparatus to contact the α-tocopherol therewith. The temperature of the coating solution was 21°C, which was the same as room temperature. The liquids remaining in the bores were removed by air flash. Then, the apparatus was aerated with dry air of 24°C for 30 minutes to remove the solvent by drying, thereby covering the membrane surface with α-tocopherol. The blood feed- and exit-side nozzles were stoppered. The resulting module was then exposed to 25 kGy electron beam to obtain a hollow fiber membrane-type blood processing apparatus having an effective membrane area of 1.5 $m^2$.

In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 33% by mass, and the content A of the hydrophilic polymer (PVP) in the whole of the hollow fiber membrane was 3% by mass.

Results of assaying its properties are shown in Table 2 below.

The obtained hollow fiber membrane-type blood processing apparatus had unfavorable blood compatibility as is evident from high LDH activity, poor productivity attributed to the high rate of misdetection in the leak test, and insufficiently stable performance under harsh conditions.

[Comparative Example 9]

The α-tocopherol concentration of the coating solution was set to 0.05% by mass, and PVP was not added. The IPA concentration was set to 57% by mass. The temperature of the coating solution was set to 21°C for injection. After drying off of the coating solution, 120 mL of an aqueous solution containing glycerin (manufactured by Wako Pure Chemical Industries, Ltd., special grade) at a concentration of 63.1% by mass was injected into the hollow fiber membranes from the open end, followed by flash with 0.3 MPa air for 10 seconds. Then, header caps were attached to both ends. The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 0.5 mg per g of the membrane, the abundance ratio B of the hydrophilic polymer (PVP) in the inner surface of the hollow fiber membrane was 33% by mass, and the content A of the hydrophilic polymer (PVP) in the whole of the hollow fiber membrane was 3% by mass.

Results of assaying its properties are shown in Table 2 below.

The obtained hollow fiber membrane-type blood processing apparatus had unfavorable blood compatibility as is evident from high LDH activity, poor productivity attributed to the high rate of misdetection in the leak test, and insufficiently stable performance under harsh conditions.

[Comparative Example 10]

120 mL of glycerin having a concentration of 63.1% by mass was injected as the coating solution into the hollow fiber membranes, followed by flash with 0.3 MPa air for 10 seconds. Then, header caps were attached to both ends. The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 0 mg per g of the membrane, the abundance ratio B of the hydrophilic polymer (PVP) in the inner surface of the hollow fiber membrane was 33% by mass, and the content A of the hydrophilic polymer (PVP) in the whole of the hollow fiber membrane was 3% by mass.

Results of assaying its properties are shown in Table 2 below.

The obtained hollow fiber membrane-type blood processing apparatus had low antioxidative capacity, unfavorable blood compatibility as is evident from high LDH activity, poor productivity attributed to the high rate of misdetection

in the leak test, and insufficiently stable performance under harsh conditions.

[Comparative Example 11]

The PVP concentration of the coating solution was set to 0.01% by mass. The IPA concentration was set to 57% by mass. The temperature of the coating solution was set to 21°C for injection. The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 6 mg per g of the membrane, the abundance ratio B of the hydrophilic polymer (PVP) in the inner surface of the hollow fiber membrane was 33% by mass, and the content A of the hydrophilic polymer (PVP) in the whole of the hollow fiber membrane was 3% by mass.

Results of assaying its properties are shown in Table 2 below.

The obtained hollow fiber membrane-type blood processing apparatus had unfavorable blood compatibility as is evident from high LDH activity, poor productivity attributed to the high rate of misdetection in the leak test, and insufficiently stable performance under harsh conditions.

[Comparative Example 12]

For the dope, the amount of PVP added was set to 10 parts by mass, and the amount of dimethylacetamide added was set to 73 parts by mass. The other conditions were the same as those of Example 1 to obtain a hollow fiber membrane-type blood processing apparatus.

In this hollow fiber membrane-type blood processing apparatus, the abundance of the fat-soluble vitamin (α-tocopherol) in the entire surface of the hollow fiber membrane was 7 mg per g of the membrane, the abundance ratio B of the hydrophilic polymer (PVP) in the inner surface of the hollow fiber membrane was 47% by mass, and the content A of the hydrophilic polymer (PVP) in the whole of the hollow fiber membrane was 12% by mass.

Results of assaying its properties are shown in Table 2 below.

The obtained hollow fiber membrane-type blood processing apparatus had insufficiently stable performance under harsh conditions, failed to produce high membrane performance, and was problematic for practical use due to the low toughness of the hollow fiber membrane, a risk of causing a leak during production or transportation, and a large amount of effluents.

[Table 1]

| | Example 1 | Example 2 | Example 3 | Example 4 | Example 5 | Example 6 | Example 7 | Example 8 | Example 9 | Example 10 |
|---|---|---|---|---|---|---|---|---|---|---|
| Abundance (mg/gHF) of fat-soluble vitamin present in entire surface of hollow fiber membrane | 7 | 0.5 | 1.5 | 18 | 25 | 6 | 6 | 6 | 6 | 6 |
| Content A (% by mass) of hydrophilic polymer in whole of hollow fiber membrane | 3 | 3 | 3 | 3 | 3 | 3 | 3 | 3 | 3 | 10 |
| Abundance ratio B (% by mass) of hydrophilic polymer in inner surface of hollow fiber membrane | 44 | 46 | 46 | 44 | 43 | 35 | 39 | 40 | 50 | 39 |
| Antioxidative capacity (mg/gHF) | ○ 5 | ○ 0.4 | ○ 1.4 | ○ 17 | ○ 24 | ○ 5 | ○ 5 | ○ 5 | ○ 5 | ○ 5 |
| LDH activity (Δabs/hr/$m^2$) | ○ 5 | ○ 45 | ○ 9 | ○ 8 | ○ 38 | ○ 78 | ○ 25 | ○ 16 | ○ 8 | ○ 25 |
| Rate (%) of misdetection in leak test | ○ 0.03 | ○ 0.30 | ○ 0.08 | ○ 0.05 | ○ 0.40 | ○ 0.09 | ○ 0.05 | ○ 0.04 | ○ 0.02 | ○ 0.05 |
| UFR before heat treatment (ml/(hr·mmHg) | 59 | 65 | 63 | 50 | 45 | 62 | 61 | 52 | 45 | 68 |
| UFR after heat treatment (ml/(hr·mmHg) | ○ 55 | ○ 60 | ○ 59 | ○ 48 | ○ 43 | ○ 57 | ○ 56 | ○ 48 | ○ 42 | ○ 63 |
| Rate (%) of change in UFR between before and after heat treatment | ○ 6.9 | ○ 7.5 | ○ 7.0 | ○ 4.0 | ○ 4.2 | ○ 8.1 | ○ 7.7 | ○ 7.0 | ○ 6.7 | ○ 7.7 |
| Residual quantity (mL) of air after priming | O 1.2 | O 1.4 | ○ 1.4 | O 1.2 | ○ 1.2 | ○ 0.1 | ○ 0.1 | O 0.3 | ○ 1.8 | ○ 0.1 |
| Toughness of hollow fiber membrane (×1000gf·%) | ○ 1.3 | ○ 1.3 | ○ 1.3 | ○ 1.3 | ○ 1.1 | O 1.3 | O 1.3 | ○ 1.3 | ○ 1.3 | ○ 1.1 |
| Effluent from hollow fiber membrane (UV-abs) | ○ 0.02 | ○ 0.02 | ○ 0.02 | ○ 0.02 | ○ 0.02 | ○ 0.02 | ○ 0.02 | ○ 0.02 | ○ 0.03 | ○ 0.09 |

(continued)

| | Example 1 | Example 2 | Example 3 | Example 4 | Example 5 | Example 6 | Example 7 | Example 8 | Example 9 | Example 10 |
|---|---|---|---|---|---|---|---|---|---|---|
| Concentration (ppm) of nitric acid ion contained in hollow fiber membrane | ○<br>3 | ○<br>3 | ○<br>3 | ○<br>3 | ○<br>1 | ○<br>3 | ○<br>2 | ○<br>6 | ○<br>8 | ○<br>3 |

[Table 2]

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
|---|---|---|---|---|---|---|---|---|---|---|---|---|
| Abundance (mg/gHF) of fat-soluble vitamin present entire surface of hollow fiber membrane | 0.4 | 27 | 6 | 6 | 6 | 7 | 0 | 6 | 0.5 | 0 | 6 | 6 |
| Content A (% by mass) of hydrophilic polymer in whole of hollow fiber membrane | 3 | 3 | 3 | 3 | 2 | 12 | 4 | 3 | 3 | 3 | 3 | 12 |
| Abundance ratio B (% by mass) of hydrophilic polymer in inner surface of hollow fiber membrane | 44 | 44 | 32 | 60 | 30 | 31 | 60 | 31 | 33 | 31 | 31 | 47 |
| Antioxidative capacity (mg/gHF) | × 0.3 | ○ 25 | ○ 5 | ○ 5 | ○ 5 | ○ 6 | × 0 | ○ 5 | ○ 5 | × 0 | ○ 5 | ○ 5 |
| LDH activity ($\Delta$abs/hr/$m^2$) | × 289 | × 267 | × 305 | ○ 8 | × 417 | × 263 | × 158 | × 388 | × 211 | × 302 | × 347 | ○ 29 |
| Rate (%) of misdetection in leak test | × 4 | × 7 | × 6 | ○ 0.01 | × 6 | × 6 | × 5 | × 6 | × 6 | × 8 | × 5 | ○ 0.01 |

(continued)

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
|---|---|---|---|---|---|---|---|---|---|---|---|---|
| UFR before heat treatment (ml/(hr·mmHg) | 65 | 42 | 62 | 45 | 50 | 64 | 67 | 61 | 63 | 70 | 62 | 58 |
| UFR after heat treatment (ml/(hr·mmHg) | × 33 | ○ 40 | × 38 | × 29 | × 29 | ○ 50 | × 34 | × 30 | ○ 87 | ○ 106 | × 32 | × 46 |
| Rate (%) of change in UFR between before and after heat treatment | × 49 | ○ 4.2 | × 38 | × 35 | × 43 | × 22 | × 49 | × 51 | × 38 | × 52 | × 48 | × 20 |
| Residual quantity (mL) of air after priming | ○ 1.1 | ○ 1.3 | ○ 0.1 | × 22 | ○ 0.1 | ○ 0 | × 24 | ○ 0 | ○ 0 | ○ 0 | ○ 0.1 | O 1.3 |
| Toughness of hollow fiber membrane (×1000gf·%) | ○ 1.3 | ○ 1.1 | ○ 1.3 | ○ 1.3 | ○ 1.4 | × 0.7 | ○ 1.3 | ○ 1.3 | ○ 1.3 | ○ 1.3 | ○ 1.3 | × 0.6 |
| Effluent from hollow fiber membrane (UV-abs) | ○ 0.02 | ○ 0.02 | ○ 0.02 | × 0.4 | × 0.2 | × 0.12 | ○ 0.02 | ○ 0.02 | ○ 0.02 | ○ 0.02 | ○ 0.02 | × 0.22 |

(continued)

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
|---|---|---|---|---|---|---|---|---|---|---|---|---|
| Concentration (ppm) of nitric acid ion contained in hollow fiber membrane | ○ 3 | ○ 3 | ○ 3 | ○ 3 | ○ 3 | ○ 3 | × 10 | ○ 3 | ○ 3 | ○ 3 | ○ 3 | ○ 3 |

In Tables 1 and 2, the unit "mg/gHF" for the abundance of the fat-soluble vitamin in the entire surface of the hollow fiber membrane means the mass (mg) of the fat-soluble vitamin in the entire surface per g of the hollow fiber membrane to be assayed.

As shown in Table 1, the hollow fiber membrane-type blood processing apparatuses of Examples 1 to 10 were all confirmed to have practically favorable antioxidative performance and blood compatibility, have high productivity attributed to the low rate of misdetection in the leak test, be easy to prime because of a sufficiently small residual quantity of air after priming, have practically sufficient toughness, carry a low risk of a leak from hollow fibers, exhibit the low elution of the hydrophilic polymer, and be more safe.

The present application is based on Japanese Patent Applications Nos. 2011-129437 (filed on June 9, 2011 to JPO) and 2011-148534 (filed on July 4, 2011 to JPO), the contents of which are incorporated herein by reference.

Industrial Applicability

The hollow fiber membrane for blood processing and the hollow fiber membrane-type blood processing apparatus of the present invention have industrial applicability as blood processing apparatuses for carrying out extracorporeal blood circulation therapy.

Reference Signs List

| | |
|---|---|
| 1 | hollow fiber membrane for blood processing |
| 1a | first passage |
| 2 | tubular container |
| 2a and 2b | port |
| 3a and 3b | resin |
| 6a and 6b | nozzle |
| 7a and 7b | header cap |
| 8 | space |
| 10 | hollow fiber membrane-type blood processing apparatus |
| 11 | second passage |
| 41 | membrane base material made of polysulfone polymer |
| 42 | hydrophilic polymer |
| 51 | hydrophilic polymer |
| 52 | thickness of hydrophilic polymer |
| 62 | additional hydrophilic polymer |
| 71 | hydrophilic polymer |
| 72 | additional hydrophilic polymer |
| 73 | thickness of diffuse layer in blood |
| 81 | fat-soluble vitamin |
| 83 | additional hydrophilic polymer |
| 91 | hydrophilic polymer |
| 92 | additional hydrophilic polymer |
| 93 | thickness of diffuse layer in blood |
| 121 | hollow fiber membrane-type blood processing apparatus |
| 122 | container containing saline |
| 124 | container |
| 125 | tube |
| 126 | head difference |
| 131 | pressure regulator |
| 132 | hollow fiber membrane-type blood processing apparatus |
| 133 | filtrate recovery container |
| 134 | test solution reservoir |
| 135 | blood pump |
| 136 | waste liquid recovery container |
| 138 | blood nozzle inlet pressure gauge |
| 137 | blood nozzle outlet pressure gauge |
| 139 | filtrate-side pressure gauge |

| | |
|---|---|
| 140 to 142 | drip chamber |
| 151 | base material made of polysulfone resin |
| 152 | hydrophilic polymer |
| 231 | dope |
| 232 | internal coagulant |
| 233 | annular slit nozzle |
| 234 | coagulation bath |
| 230 | hollow fiber membrane |
| 235 | rinsing bath |
| 236 | dryer |
| 237 | take-up roller |
| 238 | hollow fiber membrane bundle |

## Claims

**1.** A hollow fiber membrane for blood processing, comprising a hydrophilic polymer, a polysulfone-based resin, and a fat-soluble vitamin, wherein
a content A of the hydrophilic polymer, which is a mass ratio of the hydrophilic polymer to a total mass of the hydrophilic polymer and the polysulfone-based resin in a whole of the hollow fiber membrane for blood processing, is 3% by mass or larger and 10% by mass or smaller,
an abundance ratio B of the hydrophilic polymer, which is a mass ratio of the hydrophilic polymer to the total mass of the hydrophilic polymer and the polysulfone-based resin in an inner surface of the hollow fiber membrane for blood processing, is 35% by mass or larger and 50% by mass or smaller, and
an abundance of the fat-soluble vitamin in an entire surface of the hollow fiber membrane for blood processing is 0.5 mg or larger and 25 mg or smaller based on 1 g of the hollow fiber membrane.

**2.** The hollow fiber membrane for blood processing according to claim 1, wherein the hydrophilic polymer is polyvinylpyrrolidone or polyethylene glycol.

**3.** The hollow fiber membrane for blood processing according to claim 1 or 2, wherein the hollow fiber membrane for blood processing comprises 1 ppm or higher and 8 ppm or lower nitric acid ions.

**4.** A hollow fiber membrane-based blood processing apparatus provided with a hollow fiber membrane for blood processing according to any one of claims 1 to 3 inside a container.

FIG. 1

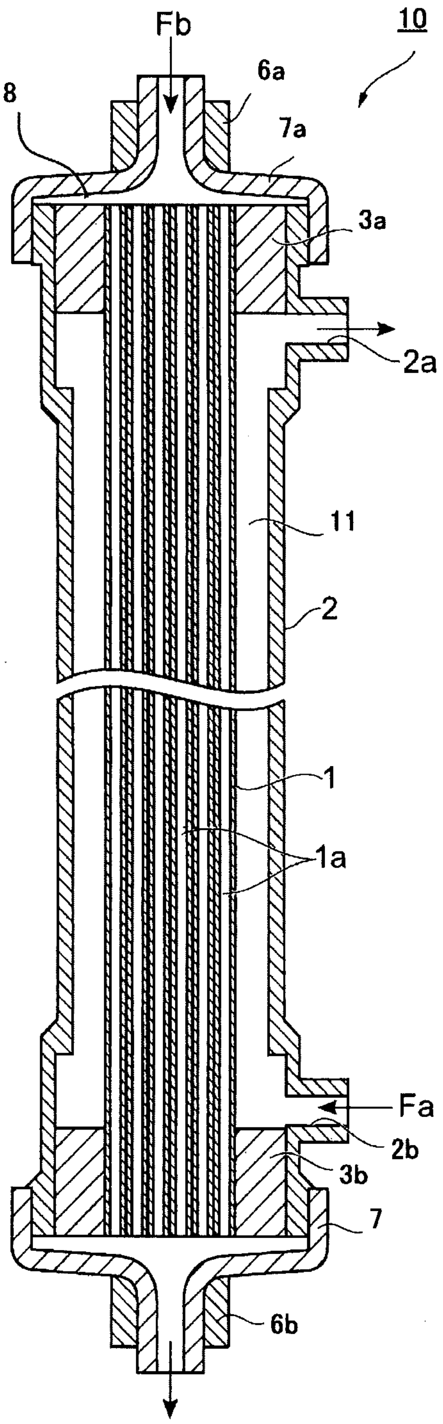

Fig. 2

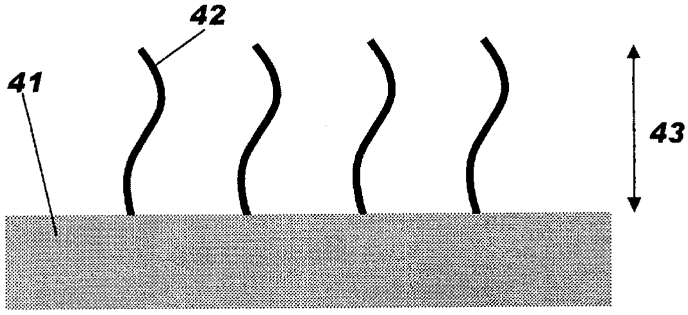

Fig. 3

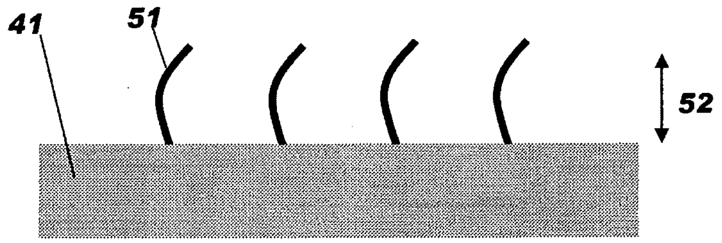

Fig. 4

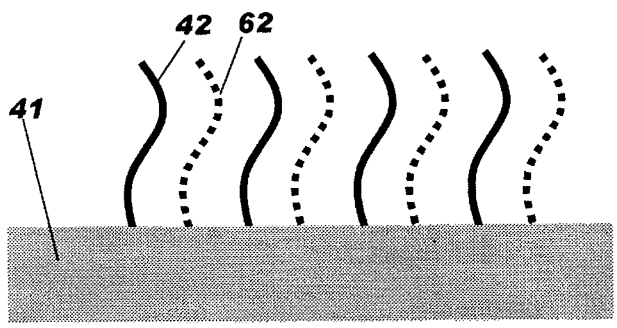

Fig. 5

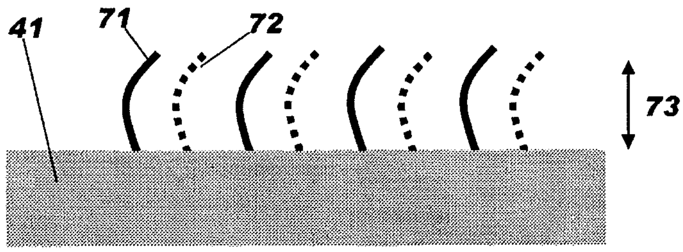

Fig. 6

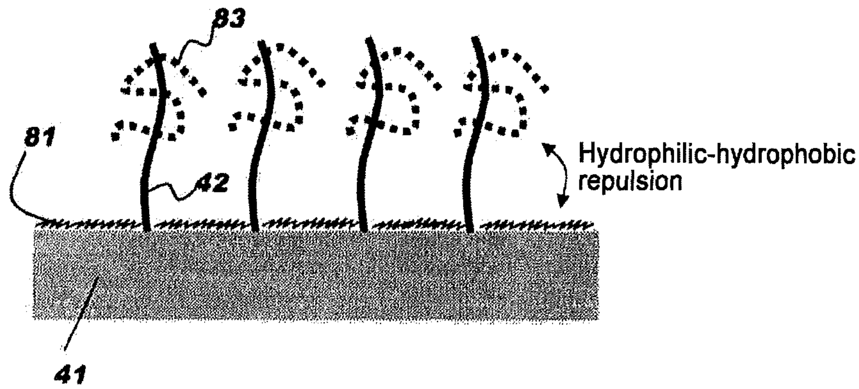

Fig. 7

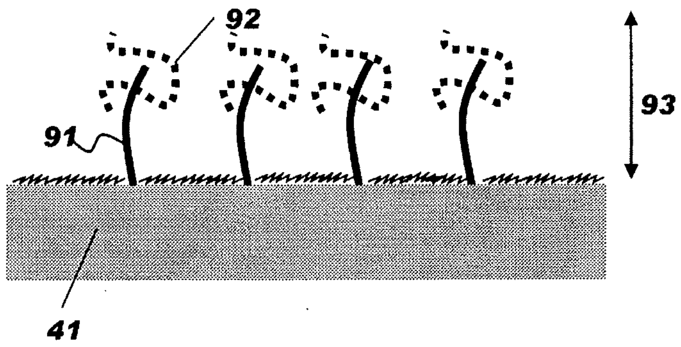

Fig. 8

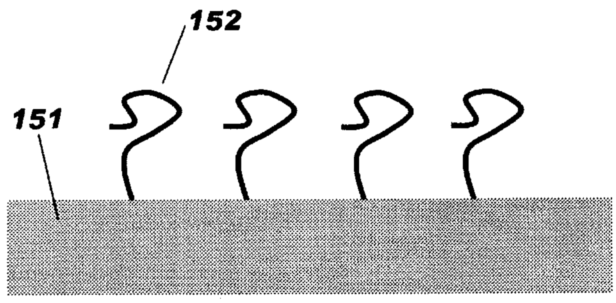

Fig. 9

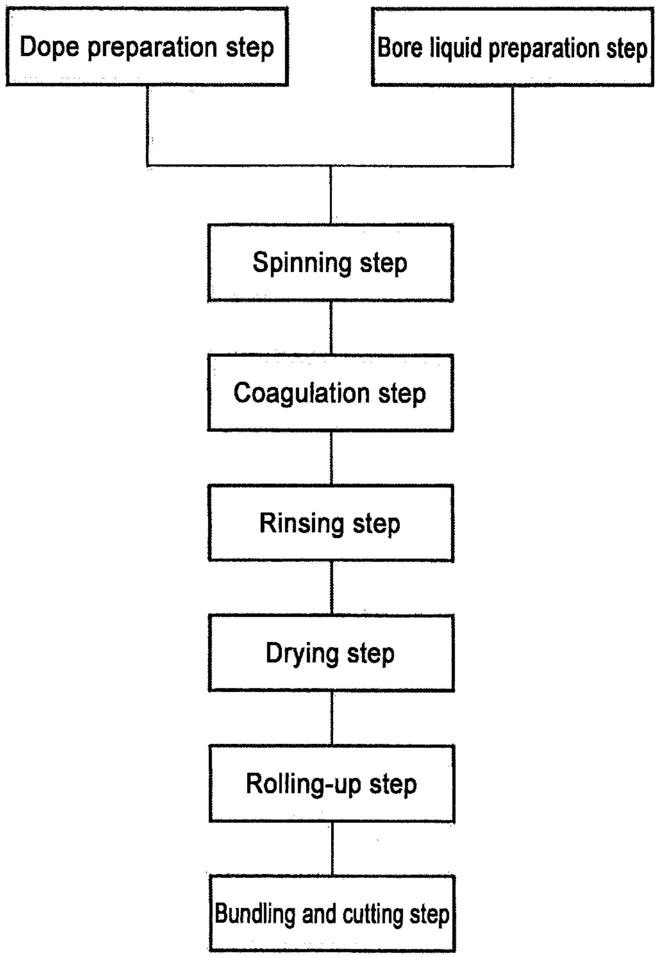
Dope preparation step
Bore liquid preparation step
Spinning step
Coagulation step
Rinsing step
Drying step
Rolling-up step
Bundling and cutting step

Fig. 10

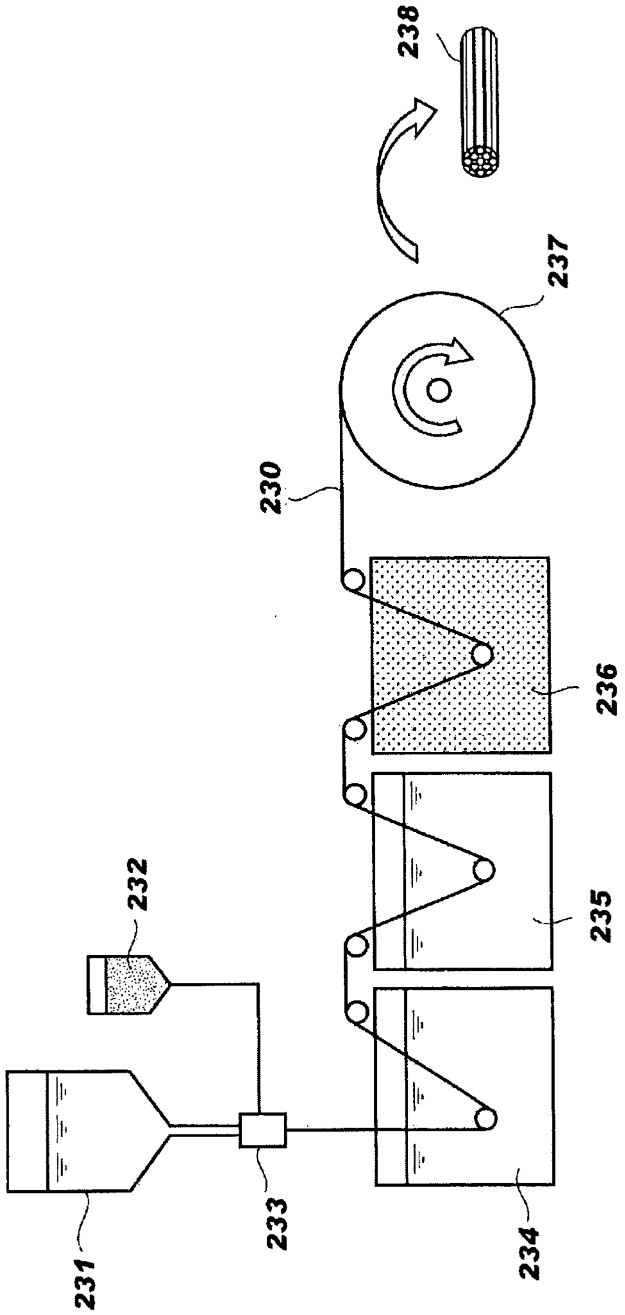

Fig. 11

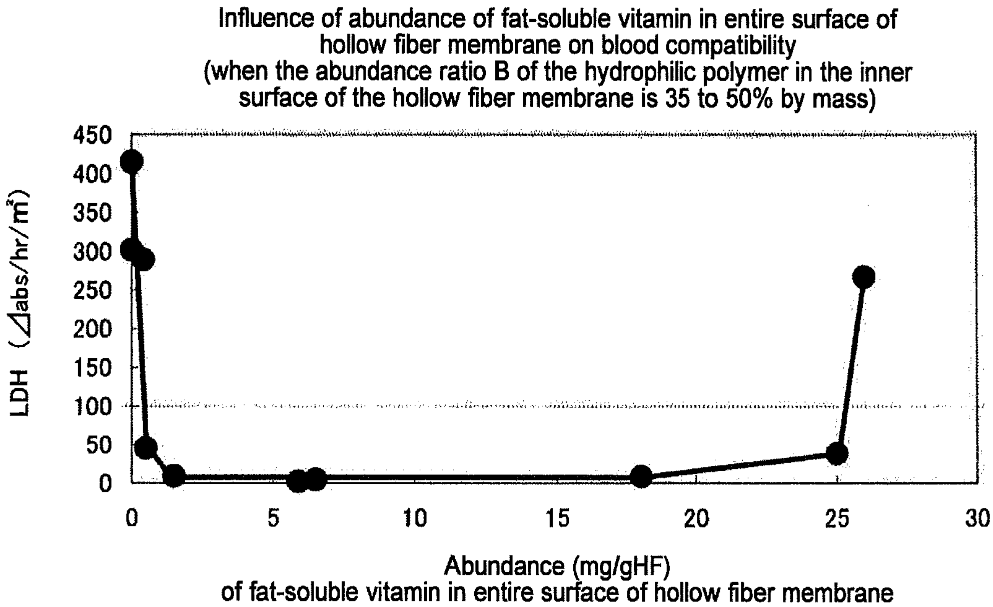
Influence of abundance of fat-soluble vitamin in entire surface of hollow fiber membrane on blood compatibility
(when the abundance ratio B of the hydrophilic polymer in the inner surface of the hollow fiber membrane is 35 to 50% by mass)
LDH (⊿abs/hr/m²)
450
400
350
300
250
200
150
100
50
0
0
5
10
15
20
25
30
Abundance (mg/gHF)
of fat-soluble vitamin in entire surface of hollow fiber membrane

Fig. 12

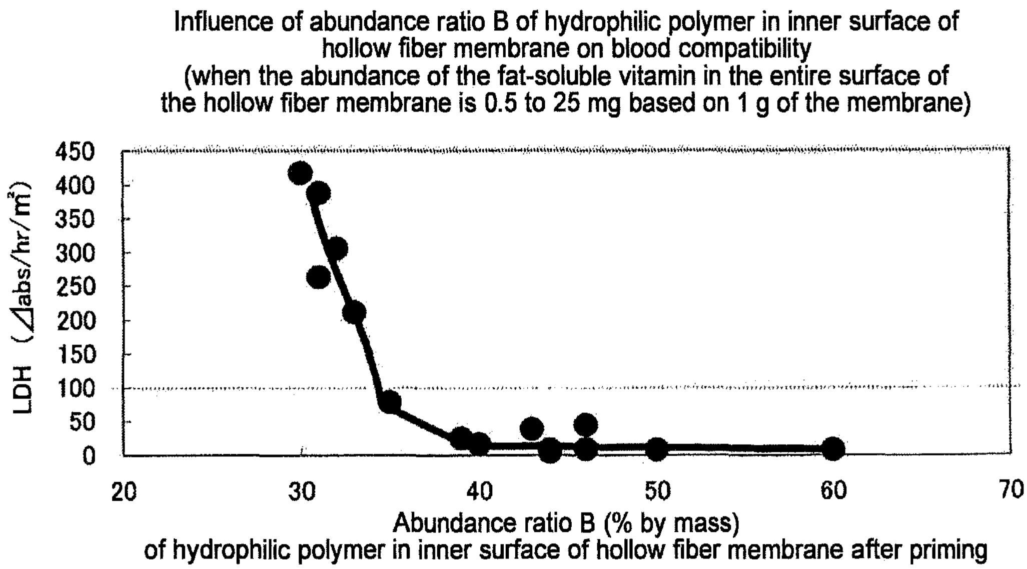
Influence of abundance ratio B of hydrophilic polymer in inner surface of hollow fiber membrane on blood compatibility
(when the abundance of the fat-soluble vitamin in the entire surface of the hollow fiber membrane is 0.5 to 25 mg based on 1 g of the membrane)
LDH (⊿abs/hr/m²)
450
400
350
300
250
200
150
100
50
0
20
30
40
50
60
70
Abundance ratio B (% by mass)
of hydrophilic polymer in inner surface of hollow fiber membrane after priming

Fig. 13

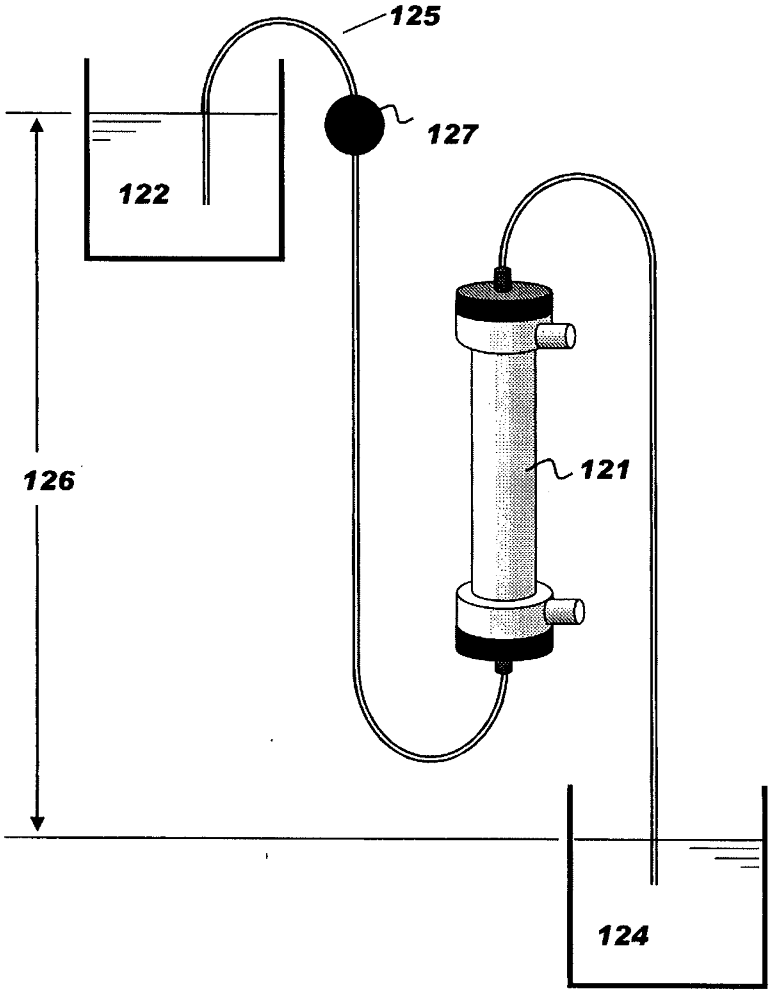

Fig. 14

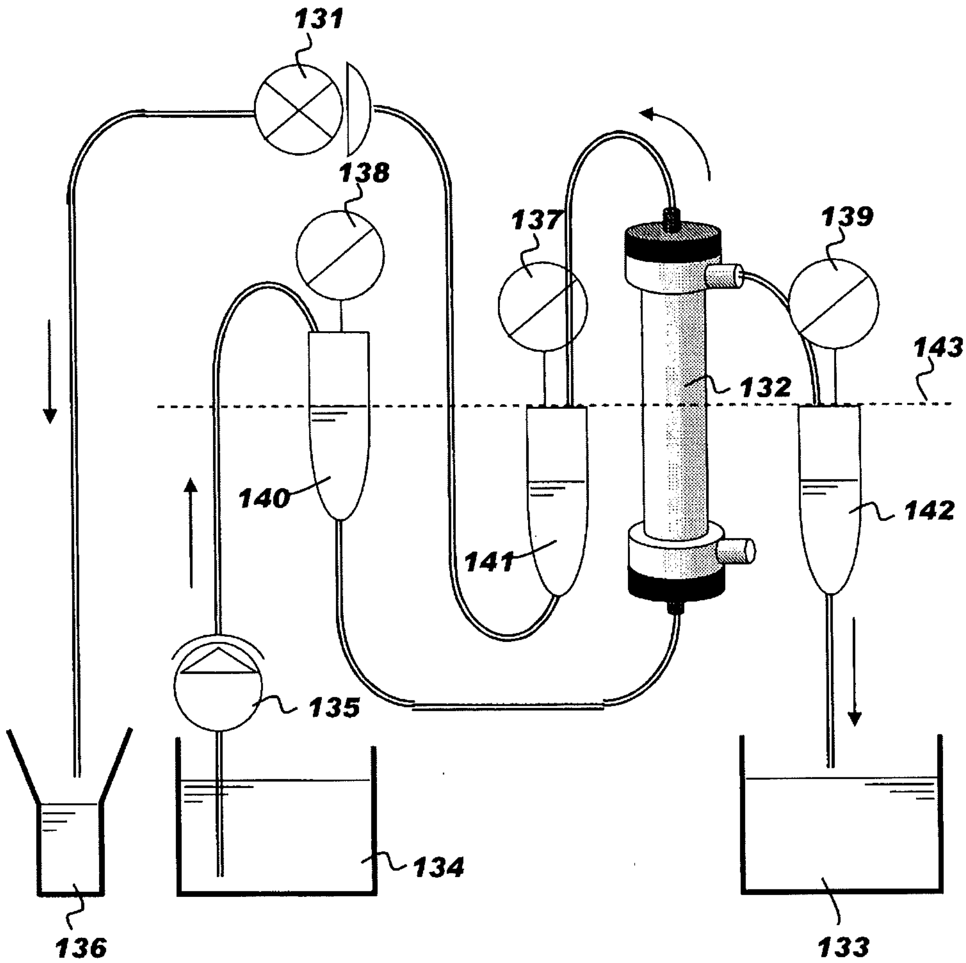

| INTERNATIONAL SEARCH REPORT | International application No. PCT/JP2012/064561 |
|---|---|

A. CLASSIFICATION OF SUBJECT MATTER
*A61M1/18*(2006.01)i, *B01D63/02*(2006.01)i, *B01D69/08*(2006.01)i, *B01D71/44*(2006.01)i, *B01D71/68*(2006.01)i, *D01F6/00*(2006.01)i, *D01F6/76*(2006.01)i, *D01F6/94*(2006.01)i

According to International Patent Classification (IPC) or to both national classification and IPC

B. FIELDS SEARCHED

Minimum documentation searched (classification system followed by classification symbols)
A61M1/18, B01D63/02, B01D69/08, B01D71/44, B01D71/68, D01F6/00, D01F6/76, D01F6/94

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2010-207583 A (Toray Industries, Inc.), 24 September 2010 (24.09.2010), entire text; all drawings & US 2005/0273031 A1 & EP 1535657 A1 & WO 2004/018085 A1 | 1-4 |
| Y | JP 2008-290009 A (Asahi Kasei Kuraray Medical Co., Ltd.), 04 December 2008 (04.12.2008), entire text; all drawings & US 2010/0133170 A1 & EP 2151273 A1 & WO 2008/146775 A1 | 1-4 |
| Y | JP 11-347117 A (Terumo Corp.), 21 December 1999 (21.12.1999), entire text; all drawings & US 6478960 B1 & EP 923955 A2 | 1-4 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 August, 2012 (29.08.12) | 11 September, 2012 (11.09.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/064561

C (Continuation). DOCUMENTS CONSIDERED TO BE RELEVANT

| Category* | Citation of document, with indication, where appropriate, of the relevant passages | Relevant to claim No. |
|---|---|---|
| A | JP 2007-215569 A (Asahi Kasei Medical Co., Ltd.), 30 August 2007 (30.08.2007), entire text; all drawings (Family: none) | 1-4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7178166 A
- JP 2006296931 A
- JP 2008093228 A
- JP 2005238096 A
- JP 2003190747 A
- JP 2009183822 A
- JP 2011129437 A
- JP 2011148534 A

### Non-patent literature cited in the description

- **ANDRULLI S et al.** *Nephron Clin Pract.,* 2010, vol. 115, c82-89
- **PANICHI V et al.** *Blood Purification,* 2011, vol. 32, 7-14